# EUROPEAN PATENT SPECIFICATION

(11) **EP 1 706 482 B1**
(45) Date of publication and mention of the grant of the patent: **22.07.2015**
(21) Application number: 05701018.3
(22) Date of filing: 18.01.2005
(51) Int. Cl.: C12N 1/21, C12P 13/04, C12P 13/08

(54) **PROCESS FOR THE PREPARATION OF L-AMINO ACIDS USING STRAINS OF THE ENTEROBACTERIACEAE FAMILY**
VERFAHREN ZUR HERSTELLUNG VON L-AMINOSÄUREN UNTER VERWENDUNG VON STÄMMEN DER FAMILIE ENTEROBACTERIACEAE
PROCEDE DE PREPARATION DE L-ACIDES AMINES A PARTIR DE SOUCHES DE LA FAMILLE DES ENTEROBACTERIACEAE

(30) Priority: 23.01.2004 DE 102004003411
(43) Date of publication of application: 04.10.2006
(73) Proprietor: Evonik Degussa GmbH, 45128 Essen (DE)
(72) Inventor: RIEPING, Mechthild, 33619 Bielefeld (DE); HERMANN, Thomas, 4200 Hajduszoboszlo (HU)
(86) International application number: PCT/EP2005/000449
(87) International publication number: WO 2005/071062

(56) References cited:
- EP-A- 1 254 957
- WO-A-02/12291
- WO-A-03/008605
- PEIST RALF ET AL: "Characterization of the aes gene of Escherichia coli encoding an enzyme with esterase activity" JOURNAL OF BACTERIOLOGY, vol. 179, no. 24, December 1997 (1997-12), pages 7679-7686, XP002325025 ISSN: 0021-9193
- RICHET E ET AL: "PURIFICATION AND PROPERTIES OF THE MALT PROTEIN THE TRANSCRIPTION ACTIVATOR OF THE ESCHERICHIA-COLI MALTOSE REGULON" JOURNAL OF BIOLOGICAL CHEMISTRY, vol. 262, no. 26, 1987, pages 12647-12653, XP002324985 ISSN: 0021-9258

## Description

### Field of the Invention

This invention relates to a process for the preparation of L-amino acids, in particular L-threonine, using recombinant microorganisms of the Enterobacteriaceae family in which the malT gene is enhanced, in particular over-expressed, and to these microorganisms.

### Background of the Invention

L-Amino acids, in particular L-threonine, are used in human medicine and in the pharmaceuticals industry, in the foodstuffs industry and very particularly in animal nutrition.

It is known that L-amino acids are prepared by fermentation of strains of Enterobacteriaceae, in particular Escherichia coli (E. coli) and Serratia marcescens. Because of their great importance, work is constantly being undertaken to improve the preparation processes. Improvements to the process can relate to fermentation measures, such as e.g. stirring and supply of oxygen, or the composition of the nutrient media, such as e.g. the sugar concentration during the fermentation, or the working up to the product form, by e.g. ion exchange chromatography, or the intrinsic output properties of the microorganism itself.

Methods of mutagenesis, selection and mutant selection are used to improve the output properties of these microorganisms. Strains which are resistant to antimetabolites, such as e.g. the threonine analogue α-amino-β-hydroxyvaleric acid (AHV), or are auxotrophic for metabolites of regulatory importance and produce L-amino acid, such as e.g. L-threonine, are obtained in this manner.

Methods of the recombinant DNA technique have also been employed for some years for improving the strain of strains of the Enterobacteriaceae family which produce L-amino acids, by amplifying individual amino acid biosynthesis genes and investigating the effect on the production. Summarizing information on the cell and molecular biology of Escherichia coli and Salmonella are to be found in Neidhardt (ed): Escherichia coli and Salmonella, Cellular and Molecular Biology, 2nd Edition, ASM Press, Washington, D.C., USA (1996).

The maltose system of Escherichia coli contains 10 genes which regulate the uptake and metabolism of maltose and maltodextrin (Boos, W. and Shuman, H.A.; Microbiology and Molecular Biology Reviews 16: 204-229 (1998)). These genes are under the control of MalT, a transcriptional activator with 901 amino acids and a size of 103 kDa. MalT belongs to a family of bacterial transactivators, the MalT or LAL family (Dannot. O.; Proceedings of the National Academy of Sciences of the United States of America 98: 435-440 (2001)). The family has the common features of a size of > 90 kDa, an ATP-binding site in the region of the N terminus and LuxR homology in the region of the C terminus. The MalT activity requires the presence of ATP and maltodextrin as an effector (Raibaud, O. and Richet, E.; Journal of Bacteriology 169: 3059-3061 (1989) and Richet, E. and Raibaud, O.; EMBO Reports 8: 981-987 (1989)). In the cell, MalT is present in non-bound form as a monomer and is converted into the oligomeric active form in the presence of ATP and maltotriose, which renders possible cooperative binding to sequences of the mal promoters (Vidal-Ingigliardi et al.; The Journal of Biological Chemistry 286: 24527-24530 (1993)).

In comparison with the positive effectors, three proteins are known which can negatively influence the MalT activity: MalK as an ATP-hydrolysing sub-unit of the maltodextrin transport system (Hofnung et al.; Genetics 76: 169-184 (1974) and Reyes, M and Shuman H. A.; Journal of Bacteriology 170: 4598-4602 (1988)). Null mutations of malK lead to a constitutive expression of the regulon, but its over-expression leads to scarcely measurable expression. A second repressor of the maltose regulon is malY.

MalY competes with MalT for the binding of maltotriose, and thus inhibits the transcriptional activity and stabilizes MalT in its inactive monomeric form (Schreiber et al.; The Journal of Biological Chemistry 35: 765-776 (2000). A third protein which inhibits MalT activity is the Aes protein, an enzyme which, plasmid-coded with its own promoter, lowers the expression of the mal gene (Peist et al.; Journal of Bacteriology 161: 1201-1208 (1985).

### Object of the Invention

The inventors had the object of providing new measures for improved preparation of L-amino acids, in particular L-threonine.

### Summary of the Invention

The invention provides recombinant microorganisms of the Enterobacteriaceae family which contain an overexpressed malT gene, which codes for the positive transcriptional activator of the maltose regulon and an overexpressed yaaU open reading frame of E. coli, which codes for a gene product annotated as a putative transport protein, wherein said yaaU open reading frame encodes the protein YaaU corresponding to Accession No. AE005181 of Fig. 3, and which produce L-threonine or L-lysine in an improved manner.

The starting point for the comparison are in each case the microorganisms which are not recombinant for the malT gene and contain no enhanced malT gene.

These microorganisms include, in particular, microorganisms of the Enterobacteriaceae family in which a polynucleotide which codes for a polypeptide of which the amino acid sequence is identical to the extent of at least 90%, in particular to the extent of at least 95%, preferably to the extent of at least 98% or at least 99%, particularly preferably to the extent of 99.7% and very particularly preferably to the extent of 100% to an amino acid sequence chosen from the group consisting of SEQ ID No. 4 and SEQ ID No. 6 is enhanced.

Amino acid sequences which are identical to those from SEQ ID No. 4 or SEQ ID No. 6 are preferred.

The microorganisms mentioned contain overexpressed polynucleotides chosen from the group consisting of:
a) polynucleotide with the nucleotide sequence SEQ ID No. 3 or SEQ ID No. 5;
b) polynucleotide with a nucleotide sequence which corresponds to SEQ ID No. 3 or SEQ ID No. 5 within the context of degeneration of the genetic code;
c) polynucleotide sequence with a sequence which hybridizes under stringent conditions with the sequence complementary to SEQ ID No. 3 or SEQ ID No. 5;
d) polynucleotide with a sequence SEQ ID No. 3 or SEQ ID No. 5 which contains neutral-function sense mutants,
where the polynucleotides code for the positive transcriptional activator of the maltose regulon.

### Detailed Description of the Invention

The microorganisms described are preferably employed.

Where L-amino acids or amino acids are mentioned in the following, this means one or more amino acids, including their salts, chosen from the group consisting of L-threonine and L-lysine. L-Threonine is particularly preferred.

The term "enhancement" in this connection describes the increase in the intracellular activity or concentration of one or more enzymes or proteins in a microorganism which are coded by the corresponding DNA, for example by increasing the number of copies of the gene or genes by at least one (1) copy, or linking a potent promoter functionally to the gene or using or a gene or allele which codes for a corresponding enzyme or protein with a high activity, and optionally combining these measures.

Alleles are in general understood as meaning alternative forms of a given gene. The forms are distinguished by differences in the nucleotide sequence.

The protein coded by a nucleotide sequence, i.e. an ORF, a gene or an allele, or the ribonucleic acid coded is in general called the gene product.

By enhancement measures the activity or concentration of the corresponding protein is in general increased by at least 10%, 25%, 50%, 75%, 100%, 150%, 200%, 300%, 400% or 500%, up to a maximum of 1,000% or 2,000%, based on that of the wild-type protein or on the activity or concentration of the protein in the microorganism or parent strain which is not recombinant for the corresponding enzyme or protein. Microorganism or parent strain which is not recombinant is understood as meaning the microorganism on which the inventive measures are carried out.

The invention provides a process for the preparation of L-threonine or L-lysine by fermentation of recombinant microorganisms of the Enterobacteriaceae family, characterized in that
a) the microorganisms of the Enterobacteriaceae family which produce the desired L-amino acids and in which the malT gene, which codes for the positive transcriptional activator of the maltose regulon, and the yaaU open reading frame of E. coli, which codes for a gene product annotated as a putative transport protein, wherein said yaaU open reading frame encodes the protein YaaU corresponding to Accession No. AE005181, or nucleotide sequences or alleles which code for the gene products MalT or YaaU are overexpressed, are cultured in a medium under conditions under which the desired L-amino acid is concentrated in the medium or in the cells of the microorganisms, and
b) the desired L-amino acid is isolated, constituents of the fermentation broth and/or the biomass in its entirety or portions (≥ 0 to 100%) thereof remaining in the product which has been isolated or being removed completely.

The recombinant microorganisms, with an overexpressed malT gene and an overexpressed yaaU open reading frame, which the present invention also provides can produce L-amino acids from glucose, sucrose, lactose, fructose, maltose, molasses, optionally starch, optionally cellulose or from glycerol and ethanol. They are representatives of the Enterobacteriaceae family chosen from the genera Escherichia, Erwinia, Providencia and Serratia. The genera Escherichia and Serratia are preferred. Of the genus Escherichia in particular the species Escherichia coli and of the genus Serratia in particular the species Serratia marcescens are to be mentioned.

Recombinant microorganisms are in general produced by transformation, transduction or conjugation, or a combination of these methods, with a vector which contains the desired gene, an allele of this gene or parts thereof and/or a promoter which enhances the expression of the gene. This promoter can be the promoter originating by enhancing mutation from the endogenous regulatory sequence upstream of the gene, or an efficient promoter that has been fused with the gene.

Suitable strains, which produce L-threonine in particular, of the genus Escherichia, in particular of the species Escherichia coli, are, for example
- Escherichia coli H4581 (EP 0 301 572)
- Escherichia coli KY10935 (Bioscience Biotechnology and Biochemistry 61(11):1877-1882 (1997)
- Escherichia coli VNIIgenetika MG442 (US-A-4278,765)
- Escherichia coli VNIIgenetika M1 (US-A-4,321,325)
- Escherichia coli VNIIgenetika 472T23 (US-A-5,631,157)
- Escherichia coli BKIIM B-3996 (US-A-5,175,107)
- Escherichia coli kat 13 (WO 98/04715)
- Escherichia coli KCCM-10132 (WO 00/09660) Suitable L-threonine-producing strains of the genus Serratia, in particular of the species Serratia marcescens, are, for example
- Serratia marcescens HNr21 (Applied and Environmental Microbiology 38(6): 1045-1051 (1979))
- Serratia marcescens TLr156 (Gene 57(2-3): 151-158 (1987))
- Serratia marcescens T-2000 (Applied Biochemistry and Biotechnology 37(3): 255-265 (1992))

Strains, from the Enterobacteriaceae family which produce L-threonine preferably have, inter alia, one or more genetic or phenotypic features chosen from the group consisting of: resistance to α-amino-β-hydroxyvaleric acid, resistance to thialysine, resistance to ethionine, resistance to α-methylserine, resistance to diaminosuccinic acid, resistance to α-aminobutyric acid, resistance to borrelidin, resistance to cyclopentane-carboxylic acid, resistance to rifampicin, resistance to valine analogues, such as, for example, valine hydroxamate, resistance to purine analogues, such as, for example, 6-dimethylaminopurine, a need for L-methionine, optionally a partial and compensatable need for L-isoleucine, a need for meso-diaminopimelic acid, auxotrophy in respect of threonine-containing dipeptides, resistance to L-threonine, resistance to threonine raffinate, resistance to L-homoserine, resistance to L-lysine, resistance to L-methionine, resistance to L-glutamic acid, resistance to L-aspartate, resistance to L-leucine, resistance to L-phenylalanine, resistance to L-serine, resistance to L-cysteine, resistance to L-valine, sensitivity to fluoropyruvate, defective threonine dehydrogenase, optionally an ability for sucrose utilization, enhancement of the threonine operon, enhancement of homoserine dehydrogenase I-aspartate kinase I, preferably of the feed back resistant form, enhancement of homoserine kinase, enhancement of threonine synthase, enhancement of aspartate kinase, optionally of the feed back resistant form, enhancement of aspartate semialdehyde dehydrogenase, enhancement of phosphoenol pyruvate carboxylase, optionally of the feed back resistant form, enhancement of phosphoenol pyruvate synthase, enhancement of transhydrogenase, enhancement of the RhtB gene product, enhancement of the RhtC gene product, enhancement of the YfiK gene product, enhancement of a pyruvate carboxylase, and attenuation of acetic acid formation.

It has been found that microorganisms of the Enterobacteriaceae family produce L-threonine and L-lysine in an improved manner after overexpression of the malT gene and the yaaU open reading frame or alleles thereof.

The nucleotide sequences of the genes of Escherichia coli belong to the prior art (see the following text references) and can also be found in the genome sequence of Escherichia coli published by Blattner et al. (Science 277: 1453-1462 (1997)). It is known that enzymes endogenous in the host (methionine aminopeptidase) can split off the N-terminal amino acid methionine.

The nucleotide sequences for the malT gene are likewise known from Salmonella typhimurium, which also belongs to the Enterobacteriaceae family.

The malT gene is described, inter alia, by the following data:
- Description:: positive transcriptional activator of the maltose regulon
- Function:: essential for transcription of the mal genes, is induced by maltotriose and ATP
- Reference:: Cole S.T. and Raibaud O.; Gene 42(2): 201-208 (1986), Richet E. and Raibaud O.; The EMBO Journal 8 (3) : 981-987 (1989), Schreiber et al.; Molecular Microbiology 35 (4) : 765-776 (2001), Schlegel et al.; The Journal of Molecular Microbiology and Biotechnology.; 4(3): 301-307 (2002)
- Accession No.:: AE000418

The nucleic acid sequences can be found in the databanks of the National Center for Biotechnology Information (NCBI) of the National Library of Medicine (Bethesda, MD, USA), the nucleotide sequence databank of the European Molecular Biologies Laboratories (EMBL, Heidelberg, Germany or Cambridge, UK) or the DNA databank of Japan (DDBJ, Mishima, Japan).

For better clarity, the known nucleotide sequence of the malT gene from Escherichia coli is to be found in SEQ ID No. 3 and the known sequence for the malT gene from Salmonella typhimurium (AE008862.1) is to be found under SEQ ID No. 5. The amino acid sequences of the proteins coded by these reading frames are shown as SEQ ID No. 4 or SEQ ID No. 6.

The genes described in the text references mentioned can be used according to the invention. Alleles of the genes which result from the degeneracy of the genetic code or due to "sense mutations" of neutral function can furthermore be used. The use of endogenous genes is preferred.

"Endogenous genes" or "endogenous nucleotide sequences" are understood as meaning the genes or alleles or nucleotide sequences present in the population of a species.

Suitable alleles of the malT gene which contain neutral-function sense mutations include, inter alia, those which lead to at most 100 or to at most 90 or to at most 80 or to at most 70 or to at most 60 or to at most 50 or at most 40 or to at most 30 or to at most 20, preferably to at most 10 or to at most 5, very particularly preferably to at most 3 or to at most 2 or to at least one (1) conservative amino acid exchange in the protein coded by them.

In the case of aromatic amino acids, conservative exchanges are referred to when phenylalanine, tryptophan and tyrosine are exchanged for one another. In the case of hydrophobic amino acids, conservative exchanges are referred to when leucine, isoleucine and valine are exchanged for one another. In the case of polar amino acids, conservative exchanges are referred to when glutamine and asparagine are exchanged for one another. In the case of basic amino acids, conservative exchanges are referred to when arginine, lysine and histidine are exchanged for one another. In the case of acidic amino acids, conservative exchanges are referred to when aspartic acid and glutamic acid are exchanged for one another. In the case of amino acids containing hydroxyl groups, conservative exchanges are referred to when serine and threonine are exchanged for one another. All other amino acid exchanges are called non-conservative amino acid exchanges.

In the same way, those nucleotide sequences which code for variants of the proteins mentioned which additionally contain a lengthening or shortening by at least one (1) amino acid on the N or C terminus can also be used. This lengthening or shortening is not more than 50, 40, 30, 20, 10, 5, 3 or 2 amino acids or amino acid radicals.

Suitable alleles also include those which code for proteins in which at least one (1) amino acid is inserted (insertion) or removed (deletion). The maximum number of such changes, called indels, can relate to 2, 3, 5, 10, 20 but in no case more than 30 amino acids.

Suitable alleles furthermore include those which are obtainable by hybridization, in particular under stringent conditions, using SEQ ID No. 3 or SEQ ID No. 5 or parts thereof, in particular the coding regions or the sequences complementary thereto.

Instructions for identifying DNA sequences by means of hybridization can be found by the expert, inter alia, in the handbook "The DIG System Users Guide for Filter Hybridization" from Boehringer Mannheim GmbH (Mannheim, Germany, 1993) and in Liebl et al. (International Journal of Systematic Bacteriology 41: 255-260 (1991)). The hybridization takes place under stringent conditions, that is to say only hybrids in which the probe and target sequence, i.e. the polynucleotides treated with the probe, are at least 80% identical are formed. It is known that the stringency of the hybridization, including the washing steps, is influenced or determined by varying the buffer composition, the temperature and the salt concentration. The hybridization reaction is in general carried out under a relatively low stringency compared with the washing steps (Hybaid Hybridisation Guide, Hybaid Limited, Teddington, UK, 1996).

A buffer corresponding to 5x SSC buffer at a temperature of approx. 50ºC - 68ºC, for example, can be employed for the hybridization reaction. Probes can also hybridize here with polynucleotides which are less than 80% identical to the sequence of the probe. Such hybrids are less stable and are removed by washing under stringent conditions. This can be achieved, for example, by lowering the salt concentration to 2x SSC and optionally subsequently 0.5x SSC (The DIG System User's Guide for Filter Hybridisation, Boehringer Mannheim, Mannheim, Germany, 1995) a temperature of approx. 50ºC - 68ºC, approx. 52ºC - 68ºC, approx. 54ºC - 68ºC, approx. 56ºC - 68ºC, approx. 58ºC - 68ºC, approx. 60ºC - 68ºC, approx. 62 - 68ºC, approx. 64ºC - 68ºC, approx. 66ºC - 68ºC being established. Temperature ranges of approx. 64ºC - 68ºC or approx. 66ºC - 68ºC are preferred. It is optionally possible to lower the salt concentration to a concentration corresponding to 0.2x SSC or 0.1x SSC. Polynucleotide fragments which are, for example, at least 80% or at least 90%, at least 91%, at least 92%, at least 93%, at least 94%, at least 95%, at least 96%, at least 97%, at least 98% or at least 99% identical to the sequence of the probe employed or to the nucleotide sequences shown in SEQ ID No. 3 or SEQ ID No. 5 can be isolated by increasing the hybridization temperature stepwise from 50ºC to 68ºC in steps of approx. 1 - 2ºC. Further instructions on hybridization are obtainable on the market in the form of so-called kits (e.g. DIG Easy Hyb from Roche Diagnostics GmbH, Mannheim, Germany, Catalogue No. 1603558). The nucleotide sequences obtained in this way code for polypeptides which are at least 90% identical, in particular identical to the extent of at least 95%, preferably to the extent of at least 98% or at least 99%, very particularly preferably to the extent of 99.8% to the amino acid sequences shown in SEQ ID No. 4 or SEQ ID No. 6.

To achieve an over-expression, for example, expression of the genes or the catalytic properties of the proteins can be increased. The two measures can optionally be combined.

Thus, for example, the number of copies of the corresponding genes can be increased, or the promoter and regulation region or the ribosome binding site upstream of the structural gene can be mutated. Expression cassettes. which are incorporated upstream of the structural gene act in the same way. By inducible promoters, it is additionally possible to increase the expression in the course of fermentative L-threonine production, and it may furthermore be advantageous also to use promoters for the gene expression which allow another gene expression with respect to time. The expression is likewise improved by measures to prolong the life of the m-RNA. Furthermore, the enzyme activity is also increased by preventing the degradation of the enzyme protein. The genes or gene constructs can either be present in plasmids with a varying number of copies, or can be integrated and amplified in the chromosome. Alternatively, an over-expression of the genes in question can furthermore be achieved by changing the composition of the media and the culture procedure.

Methods of overexpression are adequately described in the prior art - for example by Makrides et al. (Microbiological Reviews 60 (3), 512-538 (1996)). By using vectors, the number of copies is increased by at least one (1) copy.

Vectors which can be used are plasmids such as are described, for example, in US 5,538,873. Vectors which can also be used are phages, for example the phage Mu, as described in EP 0332448, or the phage lambda (λ). An increase in the number of copies can also be achieved by incorporating a further copy into a further site of the chromosome - for example into the att site of the phage λ (Yu and Court, Gene 223, 77-81 (1998)). US 5,939,307 discloses that by incorporation of expression cassettes or promoters, such as, for example, the tac promoter, trp promoter, lpp promoter or P_{L} promoter and P_{R}-promoter of the phage λ for example upstream of the chromosomal threonine operon it was possible to achieve an increase in the expression. The promoters of the phage T7, the gear-box promoters or the nar promoter can be used in the same way. Such expression cassettes or promoters can also be used, as described in EP 0 593 792, to overexpress plasmid-bound genes. By using the lacI^{Q} allele, the expression of plasmid-bound genes can in turn be controlled (Glascock and Weickert, Gene 223, 221-231 (1998)). It is furthermore possible that the activity of the promoters is increased by modification of their sequence by means of one or more nucleotide exchanges, by insertion(s) and/or deletion(s). Another gene expression with respect to time can be achieved, for example, as described by Walker et al. (Journal of Bacteriology 181: 1269-80 (1999)) by using the growth phase-dependent fis promoter.

The expert can find general instructions in this respect, inter alia, in Chang and Cohen (Journal of Bacteriology 134: 1141-1156 (1978)), in Hartley and Gregori (Gene 13: 347-353 (1981)), in Amann and Brosius (Gene 40: 183-190 (1985)), in de Broer et al. (Proceedings of the National Academy of Sciences of the United States of America 80: 21-25 (1983)), in LaVallie et al. (BIO/TECHNOLOGY 11: 187-193 (1993)), in PCT/US97/13359, in Llosa et al. (Plasmid 26: 222-224 (1991)), in Quandt and Klipp (Gene 80: 161-169 (1989)), in Hamilton et al. (Journal of Bacteriology 171: 4617-4622 (1989)), in Jensen and Hammer (Biotechnology and Bioengineering 58: 191-195 (1998)) and in known textbooks of genetics and molecular biology.

Plasmid vectors which can replicate in Enterobacteriaceae, such as e.g. cloning vectors derived from pACYC184 (Bartolomé et al.; Gene 102: 75-78 (1991)), pTrc99A (Amann et al.; Gene 69: 301-315 (1988)) or pSC101 derivatives (Vocke and Bastia; Proceedings of the National Academy of Sciences USA 80(21): 6557-6561 (1983)) can be used. A strain transformed with a plasmid vector, where the plasmid vector carries at least the malT gene or a nucleotide sequence which codes for the gene product thereof or an allele, can be employed in a process according to the invention.

The term transformation is understood as meaning the uptake of an isolated nucleic acid by a host (microorganism).

It is also possible to transfer mutations which affect the expression of the particular genes into various strains by sequence exchange (Hamilton et al.; (Journal of Bacteriology 171: 4617-4622 (1989)), conjugation or transduction.

More detailed explanations of the terms in genetics and molecular biology are found in known textbooks of genetics and molecular biology, such as, for example, the textbook by Birge (Bacterial and Bacteriophage Genetics, 4th ed., Springer Verlag, New York (USA), 2000) or the textbook by Berg, Tymoczko and Stryer (Biochemistry, 5th ed., Freeman and Company, New York (USA), 2002) or the handbook by Sambrook et al. (Molecular Cloning, A Laboratory Manual, (3 volume set), Cold Spring Harbor Laboratory Press, Cold Spring Harbor (USA), 2001).

It may furthermore be advantageous for the production of L-amino acids, in particular L-threonine, with strains of the Enterobacteriaceae family to enhance one or more enzymes of the known threonine biosynthesis pathway or enzymes of anaplerotic metabolism or enzymes for the production of reduced nicotinamide adenine dinucleotide phosphate or enzymes of glycolysis or PTS enzymes or enzymes of sulfur metabolism, in addition to the over-expression of the malT gene. The use of endogenous genes is in general preferred.

Thus, for example, at the same time one or more of the genes chosen from the group consisting of
- at least one gene of the thrABC operon which codes for aspartate kinase, homoserine dehydrogenase, homoserine kinase and threonine synthase (US-A-4,278,765),
- the pyc gene of Corynebacterium glutamicum which codes for pyruvate carboxylase (WO 99/18228),
- the pps gene which codes for phosphoenol pyruvate synthase (Molecular and General Genetics 231(2): 332-336 (1992)),
- the ppc gene which codes for phosphoenol pyruvate carboxylase (WO 02/064808),
- the pntA and pntB genes which code for subunits of pyridine transhydrogenase (European Journal of Biochemistry 158: 647-653 (1986)),
- the rhtB gene which codes for the protein which imparts homoserine resistance (EP-A-0 994 190),
- the rhtC gene which codes for the protein which imparts threonine resistance (EP-A-1 013 765),
- the thrE gene of Corynebacterium glutamicum which codes for the threonine export carrier protein (WO 01/92545),
- the gdhA gene which codes for glutamate dehydrogenase (Nucleic Acids Research 11: 5257-5266 (1983); Gene 23: 199-209 (1983)),
- the pgm gene which codes for phosphoglucomutase (WO 03/004598),
- the fba gene which codes for fructose biphosphate aldolase (WO 03/004664),
- the ptsH gene of the ptsHIcrr operon which codes for the phosphohistidine protein hexose phosphotransferase of the phosphotransferase system PTS (WO 03/004674),
- the ptsI gene of the ptsHIcrr operon which codes for enzyme I of the phosphotransferase system PTS (WO 03/004674),
- the crr gene of the ptsHIcrr operon which codes for the glucose-specific IIA component of the phosphotransferase system PTS (WO 03/004674),
- the ptsG gene which codes for the glucose-specific IIBC component (WO 03/004670),
- the lrp gene which codes for the regulator of the leucine regulon (WO 03/004665),
- the fadR gene which codes for the regulator of the fad regulon (WO 03/038106),
- the iclR gene which codes for the regulator of central intermediate metabolism
   (WO 03/038106),
- the ahpC gene of the ahpCF operon which codes for the small sub-unit of alkyl hydroperoxide reductase (WO 03/004663),
- the ahpF gene of the ahpCF operon which codes for the large sub-unit of alkyl hydroperoxide reductase (WO 03/004663),
- the cysK gene which codes for cysteine synthase A (WO 03/006666),
- the cysB gene which codes for the regulator of the cys regulon (WO 03/006666),
- the cysJ gene of the cysJIH operon which codes for the flavoprotein of NADPH sulfite reductase (WO 03/006666),
- the cysI gene of the cysJIH operon which codes for the haemoprotein of NADPH sulfite reductase (WO 03/006666),
- the cysH gene of the cysJIH operon which codes for adenylyl sulfate reductase (WO 03/006666),
- the rseA gene of the rseABC operon which codes for a membrane protein with anti-sigmaE activity (WO 03/008612),
- the rseC gene of the rseABC operon which codes for a global regulator of the sigmaE factor (WO 03/008612),
- the sucA gene of the sucABCD operon which codes for the decarboxylase sub-unit of 2-ketoglutarate dehydrogenase (WO 03/008614),
- the sucB gene of the sucABCD operon which codes for the dihydrolipoyltranssuccinase E2 sub-unit of 2-ketoglutarate dehydrogenase (WO 03/008614),
- the sucC gene of the sucABCD operon which codes for the β-sub-unit of succinyl-CoA synthetase (WO 03/008615),
- the sucD gene of the sucABCD operon which codes for the α-sub-unit of succinyl-CoA synthetase (WO 03/008615),
- the aceE gene which codes for the E1 component of the pyruvate dehydrogenase complex (WO 03/076635),
- the aceF gene which codes for the E2 component of the pyruvate dehydrogenase complex (WO 03/076635),
- the rseB gene which codes for the regulator of sigmaE factor activity (Molecular Microbiology 24(2): 355-371 (1997)), and
- the gene product of the open reading frame (ORF) yodA of Escherichia coli (Accession Number AE000288 of the National Center for Biotechnology Information (NCBI, Bethesda, MD, USA), DE10361192.4)
can be enhanced.

It may furthermore be advantageous for the production of L-amino acids, in particular L-threonine, in addition to the enhancement of the malT gene, for one or more of the genes chosen from the group consisting of
- the tdh gene which codes for threonine dehydrogenase (Journal of Bacteriology 169: 4716-4721 (1987)),
- the mdh gene which codes for malate dehydrogenase (E.C. 1.1.1.37) (Archives in Microbiology 149: 36-42 (1987)),
- the gene product of the open reading frame (orf) yjfA of Escherichia coli (Accession Number AAC77180 of the National Center for Biotechnology Information (NCBI, Bethesda, MD, USA),
   WO 02/29080)),
- the gene product of the open reading frame (orf) ytfP of Escherichia coli (Accession Number AAC77179 of the National Center for Biotechnology Information (NCBI, Bethesda, MD, USA),
   WO 02/29080)),
- the pckA gene which codes for the enzyme phosphoenol pyruvate carboxykinase (WO 02/29080),
- the poxB gene which codes for pyruvate oxidase (WO 02/36797),
- the dgsA gene which codes for the DgsA regulator of the phosphotransferase system (WO 02/081721) and is also known under the name of the mlc gene,
- the fruR gene which codes for the fructose repressor (WO 02/081698) and is also known under the name of the cra gene,
- the rpoS gene which codes for the sigma³⁸ factor
   (WO 01/05939) and is also known under the name of the katF gene, and
- the aspA gene which codes for aspartate ammonium lyase (WO 03/008603)
to be attenuated, in particular eliminated or for the expression thereof to be reduced.

The term "attenuation" in this connection describes the reduction or elimination of the intracellular activity or concentration of one or more enzymes or proteins in a microorganism which are coded by the corresponding DNA, for example by using a weaker promoter than in the microorganism or parent strain which is not recombinant for the corresponding enzyme or protein or a gene or allele which codes for a corresponding enzyme or protein with a low activity or inactivating the corresponding enzyme (protein) or the open reading frame or the gene and optionally combining these measures.

By attenuation measures, the activity or concentration of the corresponding protein is in general reduced to 0 to 75%, 0 to 50%, 0 to 25%, 0 to 10% or 0 to 5% of the activity or concentration of the wild-type protein or of the activity or concentration of the protein in the microorganism or parent strain which is not recombinant for the corresponding enzyme or protein. Microorganism or parent strain which is not recombinant is understood as meaning the microorganism on which the inventive measures are carried out.

To achieve an attenuation, for example, expression of the genes or open reading frames or the catalytic properties of the enzyme proteins can be reduced or eliminated. The two measures can optionally be combined.

The reduction in gene expression can take place by suitable culturing, by genetic modification (mutation) of the signal structures of gene expression or also by the antisense-RNA technique. Signal structures of gene expression are, for example, repressor genes, activator genes, operators, promoters, attenuators, ribosome binding sites, the start codon and terminators. The expert can find information in this respect, inter alia, for example, in Jensen and Hammer (Biotechnology and Bioengineering 58: 191-195 (1998)), in Carrier und Keasling (Biotechnology Progress 15: 58-64 (1999)), Franch and Gerdes (Current Opinion in Microbiology 3: 159-164 (2000)) and in known textbooks of genetics and molecular biology, such as, for example, the textbook by Knippers ("Molekulare Genetik", 6th edition, Georg Thieme Verlag, Stuttgart, Germany, 1995) or that by Winnacker ("Gene und Klone", VCH Verlagsgesellschaft, Weinheim, Germany, 1990).

Mutations which lead to a change or reduction in the catalytic properties of enzyme proteins are known from the prior art. Examples which may be mentioned are the works of Qiu and Goodman (Journal of Biological Chemistry 272: 8611-8617 (1997)), Yano et al. (Proceedings of the National Academy of Sciences of the United States of America 95: 5511-5515 (1998)), Wente and Schachmann (Journal of Biological Chemistry 266: 20833-20839 (1991)). Summarizing descriptions can be found in known textbooks of genetics and molecular biology, such as e.g. that by Hagemann ("Allgemeine Genetik", Gustav Fischer Verlag, Stuttgart, 1986).

Possible mutations are transitions, transversions, insertions and deletions of at least one (1) base pair or nucleotide. Depending on the effect of the amino acid exchange caused by the mutation on the enzyme activity, "missense mutations" or "nonsense mutations" are referred to. Missense mutation leads to an exchange of a given amino acid in a protein for another, this being, in particular, a non-conservative amino acid exchange. The functional capacity or activity of the protein is impaired by this means and reduced to a value of 0 to 75%, 0 to 50%, 0 to 25%, 0 to 10% or 0 to 5%. Nonsense mutation leads to a stop codon in the coding region of the gene and therefore to a premature interruption in the translation. Insertions or deletions of at least one base pair in a gene lead to frame shift mutations, which lead to incorrect amino acids being incorporated or translation being interrupted prematurely. If a stop codon is formed in the coding region as a consequence of the mutation, this also leads to a premature termination of the translation. Deletions of at least one (1) or more codons typically also lead to a complete loss of the enzyme activity.

Instructions on generation of such mutations are prior art and can be found in known textbooks of genetics and molecular biology, such as e.g. the textbook by Knippers ("Molekulare Genetik", 6th edition, Georg Thieme Verlag, Stuttgart, Germany, 1995), that by Winnacker ("Gene und Klone", VCH Verlagsgesellschaft, Weinheim, Germany, 1990) or that by Hagemann ("Allgemeine Genetik", Gustav Fischer Verlag, Stuttgart, 1986).

Suitable mutations in the genes can be incorporated into suitable strains by gene or allele replacement.

A common method is the method, described by Hamilton et al. (Journal of Bacteriology 171: 4617-4622 (1989)), of gene replacement with the aid of a conditionally replicating pSC101 derivative pMAK705. Other methods described in the prior art, such as, for example, that of Martinez-Morales et al. (Journal of Bacteriology 181: 7143-7148 (1999)) or that of Boyd et al. (Journal of Bacteriology 182: 842-847 (2000)), can likewise be used.

It is also possible to transfer mutations in the particular genes or mutations which affect expression of the particular genes or open reading frames into various strains by conjugation or transduction.

In addition to overexpression of the malT gene and the yaaU open reading frame it may furthermore be advantageous for the production of L-amino acids, in particular L-threonine, to eliminate undesirable side reactions (Nakayariza: "Breeding of Amino Acid Producing Microorganisms", in: Overproduction of Microbial Products, Krumphanzl, Sikyta, Vanek (eds.), Academic Press, London, UK, 1982).

The microorganisms produced according to the invention can be cultured in the batch process (batch culture), in the fed batch (feed process), in the repeated fed batch process (repetitive feed process) or in a continuous process (DE102004028859.3 or US5,763,230). A summary of known culture methods is described in the textbook by Chmiel (Bioprozesstechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/ Wiesbaden, 1994)).

The culture medium to be used must meet the requirements of the particular strains in a suitable manner. Descriptions of culture media for various microorganisms are contained in the handbook "Manual of Methods for General Bacteriology" of the American Society for Bacteriology (Washington D.C., USA, 1981).

Sugars and carbohydrates, such as e.g. glucose, sucrose, lactose, fructose, maltose, molasses, starch and optionally cellulose, oils and fats, such as e.g. soya oil, sunflower oil, groundnut oil and coconut fat, fatty acids, such as e.g. palmitic acid, stearic acid and linoleic acid, alcohols, such as e.g. glycerol and ethanol, and organic acids, such as e.g. acetic acid, can be used as the source of carbon. These substances can be used individually or as a mixture.

Organic nitrogen-containing compounds, such as peptones, yeast extract, meat extract, malt extract, corn steep liquor, soya bean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture.

Phosphoric acid, potassium dihydrogen phosphate or dipotassium hydrogen phosphate or the corresponding sodium-containing salts can be used as the source of phosphorus. The culture medium must furthermore comprise salts of metals, such as e.g. magnesium sulfate or iron sulfate, which are necessary for growth. Finally, essential growth substances, such as amino acids and vitamins, can be employed in addition to the abovementioned substances. Suitable precursors can moreover be added to the culture medium. The starting substances mentioned can be added to the culture in the form of a single batch, or can be fed in during the culture in a suitable manner.

The fermentation is in general carried out at a pH of 5.5 to 9.0, in particular 6.0 to 8.0. Basic compounds, such as sodium hydroxide, potassium hydroxide, ammonia or aqueous ammonia, or acid compounds, such as phosphoric acid or sulfuric acid, can be employed in a suitable manner to control the pH of the culture. Antifoams, such as e.g. fatty acid polyglycol esters, can be employed to control the development of foam. Suitable substances having a selective action, e.g. antibiotics, can be added to the medium to maintain the stability of plasmids. To maintain aerobic conditions, oxygen or oxygen-containing gas mixtures, such as e.g. air, are introduced into the culture. The temperature of the culture is usually 25ºC to 45ºC, and preferably 30ºC to 40ºC. Culturing is continued until a maximum of L-amino acids or L-threonine has formed. This target is usually reached within 10 hours to 160 hours.

The analysis of L-amino acids can be carried out by anion exchange chromatography with subsequent ninhydrin derivatization, as described by Spackman et al. (Analytical Chemistry, 30: 1190-1206 (1958)) or it can be carried out by reversed phase HPLC, as described by Lindroth et al. (Analytical Chemistry 51: 1167-1174 (1979)).

The process according to the invention is used for the fermentative preparation of L-amino acids, such as, for example, L-threonine, L-isoleucine, L-valine, L-methionine, L-homoserine, L-tryptophan and L-lysine, in particular L-threonine.

The following microorganism has been deposited at the Deutsche Sammlung fur Mikroorganismen und Zellkulturen (DSMZ = German Collection of Microorganisms and Cell Cultures, Braunschweig, Germany) in accordance with the Budapest Treaty:

### • Escherichia coli strain E. coli MG442 as DSM 16574.

The present invention is explained in more detail in the following with the aid of embodiment examples.

The minimal (M9) and complete media (LB) for Escherichia coli used are described by J.H. Miller (A Short Course in Bacterial Genetics (1992), Cold Spring Harbor Laboratory Press). The isolation of plasmid DNA from Escherichia coli and all techniques of restriction, ligation, Klenow and alkaline phosphatase treatment are carried out by the method of Sambrook et al. (Molecular Cloning - A Laboratory Manual (1989) Cold Spring Harbor Laboratory Press). Unless described otherwise, the transformation of Escherichia coli is carried out by the method of Chung et al. (Proceedings of the National Academy of Sciences of the United States of America 86: 2172-2175 (1989)).

The incubation temperature for the preparation of strains and transformants is 37ºC.

### Example 1

### Construction of the expression plasmid pTrc99AmalT

The malT gene from E. coli K12 is amplified using the polymerase chain reaction (PCR) and synthetic oligonucleotides. Starting from the nucleotide sequence of the malT gene in E. coli K12 MG1655 (Accession Number AE000418, Blattner et al. (Science 277: 1453-1474 (1997)), PCR primers are synthesized (MWG Biotech, Ebersberg, Germany). The primers contain sequences for restriction enzymes which are marked by underlining in the nucleotide sequence shown below. The primer malT1 contains the restriction cleavage site for XabI, the primer ma1T2 contains that for HindIII.
malTl:
   5' -CCTCATTCTAGACAGTGAAGTGATTAA-3' (SEQ ID No. 1)
malT2:
   5' -GGCGCGTTATCAAGCTTAACTTACAC- 3' (SEQ ID No. 2)

The chromosomal E. coli K12 MG1655 DNA employed for the PCR is isolated according to the manufacturers instructions with "Qiagen Genomic-tips 100/G" (QIAGEN, Hilden, Germany). A DNA fragment approx. 2,755 bp in size can be amplified with the specific primers under standard PCR conditions (Innis et al. (1990) PCR Protocols. A Guide to Methods and Applications, Academic Press) with Vent-DNA polymerase (New England BioLabs, Frankfurt, Germany) (SEQ ID No. 3).

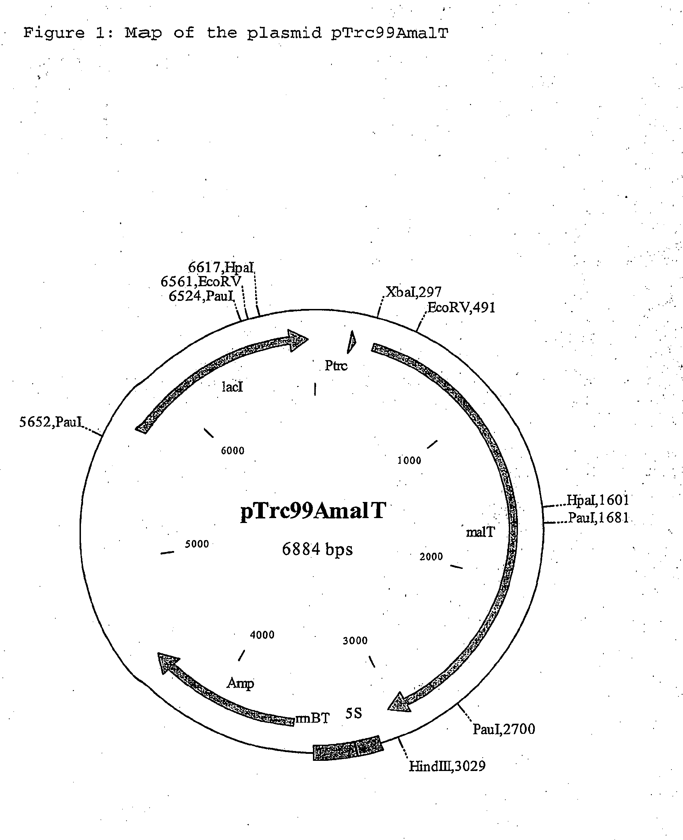

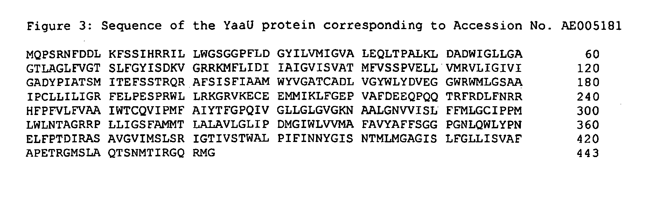

The amplified malT fragment is restricted with the restriction enzymes HindIII and XbaI and after purification (Purification Kit, QIAGEN, Hilden, Germany) checked in a 0.8% agarose gel. The vector pTrc99A (Pharmacia Biotech, Uppsala, Sweden) is cleaved with the enzymes HindIII and XbaI and ligation is carried out with the restricted malT fragment. The E. coli strain TOP10 One Shot^{®} (TOPO TA Cloning Kit, Invitrogen, Groningen, The Netherlands) is transformed with the ligation batch and plasmid-carrying cells are selected on LB agar, to which 50 µg/ml ampicillin is added. Successful cloning can be demonstrated after plasmid DNA isolation by control cleavage with the enzymes EcoRV and PauI. The plasmid is called pTrc99Ama1T (figure 1).

### Example 2

### Preparation of L-threonine with the strain

### MG442/pTrc99AmalT

The L-threonine-producing E. coli strain MG442 is described in the patent specification US-A-4,278,765 and deposited as CMIM B-1628 at the Russian National Collection for Industrial Microorganisms (VKPM, Moscow, Russia) and as DSM 16574 at the Deutsche Sammlung für Mikroorganismen und Zellkulturen (DSMZ, Braunschweig, Germany) in accordance with the Budapest Treaty.

The strain MG442 is transformed with the expression plasmid pTrc99AmalT described in example 1 and with the vector pTrc99A and plasmid-carrying cells are selected on LB agar with 50 µg/ml ampicillin. Successful transformations can be demonstrated after plasmid DNA isolation by control cleavages with the enzymes HpaI, HindIII/XbaI and EcoRV. The strains MG442/pTrc99AmalT and MG442/pTrc99A are formed in this manner. Selected individual colonies are then multiplied further on minimal medium with the following composition: 3.5 g/l Na₂HPO₄*2H₂O, 1.5 g/l KH₂PO₄, 1 g/l NH₄Cl, 0.1 g/l MgSO₄*7H₂O, 2 g/l glucose, 20 g/l agar, 50 mg/l ampicillin. The formation of L-threonine is checked in batch cultures of 10 ml contained in 100 ml conical flasks. For this, 10 ml of preculture medium of the following composition: 2 g/l yeast extract, 10 g/l (NH₄)₂5O₄, 1 g/l KH₂PO₄, 0.5 g/l MgSO₄*7H₂O, 15 g/l CaCO₃, 20 g/l glucose, 50 mg/l ampicillin are inoculated and the batch is incubated for 16 hours at 37ºC and 180 rpm on an ESR incubator from Kühner AG (Birsfelden, Switzerland).

250 µl portions of this preculture are transinoculated into 10 ml of production medium (25 g/l (NH₄)₂SO₄, 2 g/l KH₂PO₄, 1 g/l MgSO₄*7H₂O, 0.03 g/l FeSO₄*7H₂O, 0.018 g/l MnSO₄*1H₂O, 30 g/l CaCO₃, 20 g/l glucose, 50 mg/l ampicillin) and the batch is incubated for 48 hours at 37ºC. For complete induction of the expression of the malT gene, 100 mg/l isopropyl β-D-thiogalactopyranoside (IPTG) are added in parallel batches. The formation of L-threonine by the starting strain MG442 is investigated in the same manner, but no addition of ampicillin to the medium takes place. After the incubation the optical density (OD) of the culture suspension is determined with an LP2W photometer from Dr. Lange (Düsseldorf, Germany) at a measurement wavelength of 660 nm.

The concentration of L-threonine formed is then determined in the sterile-filtered culture supernatant with an amino acid analyzer from Eppendorf-BioTronik (Hamburg, Germany) by ion exchange chromatography and post-column reaction with ninhydrin detection.

The result of the experiment is shown in Table 1.

**Table 1**

| Strain | Additives | OD (660 nm) | L-Threonine g/l |
|---|---|---|---|
| MG442 | - | 5.6 | 1.4 |
| MG442/pTrc99A | - | 3.8 | 1.3 |
| MG442/pTrc99AmalT | - | 5.9 | 2.1 |
| MG442/pTrc99AmalT | IPTG | 7.2 | 2.4 |

### Brief description of the Figure:

Figure 1: Map of the plasmid pTrc99AmalT containing the malT gene.

The length data are to be understood as approx. data. The abbreviations and designations used have the following meaning:
- Amp: ampicillin resistance gene
- lacI: gene for the repressor protein of the trc promoter
- Ptrc: trc promoter region, IPTG-inducible
- malT: coding region of the malT gene
- 5S: 5S rRNA region
- rrnBT: rRNA terminator region

The abbreviations for the restriction enzymes have the following meaning
- EcoRV: restriction endonuclease from Escherichia coli B945
- HindIII: restriction endonuclease from Haemophilus influenzae R_{C}
- HpaI: restriction endonuclease from Haemophilus parainfluenzae
- PauI: restriction endonuclease from Paracoccus alcaliphilus
- XbaI: restriction endonuclease from Xanthomonas campestris

## Claims

1. Recombinant microorganisms of the Enterobacteriaceae family which contain an overexpressed malT gene, which codes for the positive transcriptional activator of the maltose regulon, and an overexpressed yaaU open reading frame of E. coli, which codes for a gene product annotated as a putative transport protein, wherein said yaaU open reading frame encodes the protein YaaU corresponding to Accession No. AE005181, of Fig. 3 and which produce L-threonine or L-lysine in an improved manner.

2. Microorganisms according to claim 1 in which a polynucleotide which codes for a polypeptide of which the amino acid sequence is identical to the extent of at least 90% to an amino acid sequence chosen from the group consisting of SEQ ID No. 4 and SEQ ID No. 6 is overexpressed.

3. Microorganisms according to claim 2, wherein they contain an overexpressed polynucleotide corresponding to the malT gene chosen from the group consisting of:
a) polynucleotide with the nucleotide sequence from SEQ ID No. 3 or SEQ ID No. 5;
b) polynucleotide with a nucleotide sequence which corresponds to SEQ ID No. 3 or SEQ ID No. 5 within the context of degeneration of the genetic code;
c) polynucleotide sequence with a sequence which hybridizes under stringent conditions with the sequence complementary to SEQ ID No. 3 or SEQ ID No. 5;
d) polynucleotide with a sequence SEQ ID No. 3 or SEQ ID No. 5 which contains neutral-function sense mutants.

4. Microorganisms according to claim 2, wherein the polypeptide has an amino acid sequence which is identical to the extent of at least 95% to one of the sequences chosen from the group consisting of SEQ ID No. 4 and SEQ ID No. 6.

5. Microorganisms according to claim 2, wherein the polypeptide has the amino acid sequence which is 100% identical to that from SEQ ID No. 4 or SEQ ID No. 6.

6. Microorganisms according to claims 1 to 5, wherein they are produced by transformation, transduction or conjugation, or a combination of these methods, with a vector which contains the malT gene and/or the yaaU open reading frame, an allele of this gene/open reading frame or parts thereof and/or a promoter.

7. Microorganisms according to claim 6, in which the number of copies of the malT gene and/or the yaaU open reading frame or of the alleles is increased by at least 1.

8. Microorganisms according to claim 7, wherein the increase in the number of copies of the malT gene and/or the yaaU open reading frame by at least 1 is effected by integration of the gene/ORF or the alleles into the chromosome of the microorganisms.

9. Microorganisms according to claim 7, wherein the increase in the number of copies of the malT gene and/or the yaaU open reading frame by at least 1 is achieved by a vector which replicates extrachromosomally.

10. Microorganisms according to claims 1 or 2, wherein, to achieve the overexpression,
a) the promoter and regulation region or the ribosome-binding site upstream of the malT gene and/or the yaaU open reading frame is mutated, or
b) expression cassettes or promoters are incorporated upstream of the malT gene and/or the yaaU open reading frame.

11. Microorganisms according to claims 1 or 2, wherein the malT gene and/or the yaaU open reading frame is/are under the control of a promoter which leads to overexpression of the gene/ORF.

12. Microorganisms according to claims 1 to 11, wherein the concentration or activity of the malT and the yaaU gene product (protein) is increased by at least 10%, based on the activity or concentration of the gene product in the microorganism or parent strain which is not recombinant for the malT gene and the yaaU open reading frame, by the overexpression of the malT gene and the yaaU open reading frame.

13. Microorganisms according to claim 1, wherein the microorganisms are chosen from the genera Escherichia, Erwinia, Providencia and Serratia.

14. Process for the preparation of L-threonine or L-lysine by fermentation of recombinant microorganisms of the Enterobacteriaceae family, wherein
a) the microorganisms which produce the desired L-amino acid and in which the malT gene, which codes for the positive transcriptional activator of the maltose regulon, and the yaaU open reading frame of E. coli, which codes for a gene product annotated as a putative transport protein, wherein said yaaU open reading frame encodes the protein YaaU corresponding to Accession No. AE005181, of Fig. 3 or nucleotide sequences or alleles which code for the gene products MalT or YaaU are overexpressed, are cultured in a medium under conditions under which the desired L-amino acid is concentrated in the medium or in the cells, and
b) the desired L-amino acid is isolated, constituents of the fermentation broth and/or the biomass in its entirety or portions (≥ 0 to 100%) thereof remaining in the product which has been isolated or being completely removed.

15. Process according to claim 14, wherein the microorganisms according to claims 1 to 13 are employed.

16. Process according to claim 14 or 15, wherein, for the preparation of L-threonine, microorganisms of the Enterobacteriaceae family in which additionally at the same time one or more of the genes chosen from the group consisting of:
16.1 at least one gene of the thrABC operon which codes for aspartate kinase, homoserine dehydrogenase, homoserine kinase and threonine synthase,
16.2 the pyc gene of Corynebacterium glutamicum which codes for pyruvate carboxylase,
16.3 the pps gene which codes for phosphoenol pyruvate synthase,
16.4 the ppc gene which codes for phosphoenol pyruvate carboxylase,
16.5 the pntA and pntB genes which code for subunits of pyridine transhydrogenase,
16.6 the rhtB gene which codes for the protein which imparts homoserine resistance,
16.7 the rhtC gene which codes for the protein which imparts threonine resistance,
16.8 the thrE gene of Corynebacterium which codes for the threonine export carrier protein,
16.9 the gdhA gene which codes for glutamate dehydrogenase,
16.10 the pgm gene which codes for phosphoglucomutase,
16.11 the fba gene which codes for fructose biphosphate aldolase,
16.12 the ptsH gene which codes for the phosphohistidine protein hexose phosphotransferase,
16.13 the ptsI gene which codes for enzyme I of the phosphotransferase system,
16.14 the crr gene which codes for the glucose-specific IIA component,
16.15 the ptsG gene which codes for the glucose-specific IIBC component,
16.16 the lrp gene which codes for the regulator of the leucine regulon,
16.17 the fadR gene which codes for the regulator of the fad regulon,
16.18 the iclR gene which codes for the regulator of central intermediate metabolism,
16.19 the ahpC gene which codes for the small sub-unit of alkyl hydroperoxide reductase,
16.20 the ahpF gene which codes for the large sub-unit of alkyl hydroperoxide reductase,
16.21 the cysK gene which codes for cysteine synthase A,
16.22 the cysB gene which codes for the regulator of the cys regulon,
16.23 the cysJ gene which codes for the flavoprotein of NADPH sulfite reductase,
16.24 the cysI gene which codes for the haemoprotein of NADPH sulfite reductase,
16.25 the cysH gene which codes for adenylyl sulfate reductase,
16.26 the rseA gene which codes for a membrane protein with anti-sigmaE activity,
16.27 the rseC gene which codes for a global regulator of the sigmaE factor,
16.28 the sucA gene which codes for the decarboxylase sub-unit of 2-ketoglutarate dehydrogenase,
16.29 the sucB gene which codes for the dihydrolipoyltranssuccinase E2 sub-unit of 2-ketoglutarate dehydrogenase,
16.30 the sucC gene which codes for the β-sub-unit of succinyl-CoA synthetase,
16.31 the sucD gene which codes for the α-sub-unit of succinyl-CoA synthetase,
16.32 the aceE gene which codes for the E1 component of the pyruvate dehydrogenase complex,
16.33 the aceF gene which codes for the E2 component of the pyruvate dehydrogenase complex,
16.34 the rseB gene which codes for the regulator of sigmaE factor activity, and
16.35 the gene product of the open reading frame (ORF) yodA of Escherichia coli,
is or are overexpressed, are fermented.

17. Process according to claim 14 or 15, wherein, for the preparation of L-threonine, microorganisms of the Enterobacteriaceae family in which additionally at the same time one or more of the genes chosen from the group consisting of:
17.1 the tdh gene which codes for threonine dehydrogenase,
17.2 the mdh gene which codes for malate dehydrogenase,
17.3 the gene product of the open reading frame (ORF) yjfA of Escherichia coli,
17.4 the gene product of the open reading frame (ORF) ytfP of Escherichia coli,
17.5 the pckA gene which codes for phosphoenol pyruvate carboxykinase,
17.6 the poxB gene which codes for pyruvate oxidase,
17.7 the dgsA gene which codes for the DgsA regulator of the phosphotransferase system,
17.8 the fruR gene which codes for the fructose repressor,
17.9 the rpoS gene which codes for the sigma³⁸ factor, and
17.10 the aspA gene which codes for aspartate ammonium lyase
is or are attenuated or eliminated, are fermented.

## Patentansprüche

1. Rekombinante Mikroorganismen aus der Familie Enterobacteriaceae, die ein überexprimiertes malT-Gen, das für den positiven Transkriptionsaktivator des Maltose-Regulons codiert, und einen überexprimierten offenen yaaU-Leserahmen von E. coli enthalten, der für ein Genprodukt codiert, das als putatives Transportprotein bezeichnet wird, wobei der offene yaaU-Leserahmen das Protein YaaU codiert, das der Zugangs-Nr. AE005181 der Fig. 3 entspricht, die L-Threonin oder L-Lysin auf verbesserte Weise produzieren.

2. Mikroorganismen nach Anspruch 1, in denen ein Polynukleotid, das ein Polypeptid codiert, dessen Aminosäuresequenz in einem Ausmaß von mindestens 90% identisch zu einer Aminosäuresequenz ist, die aus der Gruppe, bestehend aus SEQ ID NO: 4 und SEQ ID NO: 6, ausgewählt wird, überexprimiert wird.

3. Mikroorganismen nach Anspruch 2, wobei diese ein überexprimiertes Polynukleotid enthalten, das dem malT-Gen entspricht, ausgewählt aus der Gruppe, bestehend aus:
a) Polynukleotid mit der Nukleotidsequenz von SEQ ID NO: 3 oder SEQ ID NO: 5;
b) Polynukleotid mit einer Nukleotidsequenz, die SEQ ID NO: 3 oder SEQ ID NO: 5 im Rahmen der Degeneration des genetischen Codes entspricht;
c) Polynukleotidsequenz mit einer Sequenz, die unter stringenten Bedingungen mit der zu SEQ ID NO: 3 oder SEQ ID NO: 5 komplementären Sequenz hybridisiert;
d) Polynukleotid mit einer Sequenz SEQ ID NO: 3 oder SEQ ID NO: 5, das Sense-Mutanten mit neutraler Funktion enthält.

4. Mikroorganismen nach Anspruch 2, wobei das Polypeptid eine Aminosäuresequenz hat, die in einem Ausmaß von mindestens 95% identisch zu einer der Sequenzen ist, die aus der Gruppe, bestehend aus SEQ ID NO: 4 und SEQ ID NO: 6, ausgewählt werden.

5. Mikroorganismen nach Anspruch 2, wobei das Polypeptid die Aminosäuresequenz hat, die 100% identisch zu derjenigen von SEQ ID NO: 4 und SEQ ID NO: 6 ist.

6. Mikroorganismen nach den Ansprüchen 1 bis 5, wobei sie durch Transformation, Transduktion oder Konjugation oder eine Kombination dieser Verfahren mit einem Vektor, der das malT-Gen und/oder den offenen yaaU-Leserahmen, ein Allel dieses Gens/offenen Leserahmens oder Teile davon und/oder einen Promotor enthält, hergestellt werden.

7. Mikroorganismen nach Anspruch 6, wobei die Anzahl der Kopien des malT-Gens und/oder des offenen yaaU-Leserahmens oder der Allele um mindestens 1 erhöht ist.

8. Mikroorganismen nach Anspruch 7, wobei die Erhöhung in der Anzahl der Kopien des malT-Gens und/oder des offenen yaaU-Leserahmens um mindestens 1 durch Integration des Gens/ORF oder der Allele in das Chromosom der Mikroorganismen herbeigeführt wird.

9. Mikroorganismen nach Anspruch 7, wobei die Erhöhung in der Anzahl der Kopien des malT-Gens und/oder des offenen yaaU-Leserahmens um mindestens 1 durch einen Vektor, der extrachromosomal repliziert, erreicht wird.

10. Mikroorganismen nach den Ansprüchen 1 oder 2, wobei zum Erzielen der Überexpression
a) der Promotor und die Regulationsregion oder die Ribosomenbindungsstelle stromaufwärts des malT-Gens und/oder des offenen yaaU-Leserahmens mutiert wird oder
b) Expressionskassetten oder Promotoren stromaufwärts des malT-Gens und/oder des offenen yaaU-Leserahmens eingebracht werden.

11. Mikroorganismen nach den Ansprüchen 1 oder 2, wobei das malT-Gen und/oder der offene yaaU-Leserahmen unter der Kontrolle eines Promotors steht/stehen, der zur Überexpression des Gens/ORF führt.

12. Mikroorganismen nach den Ansprüchen 1 bis 11, wobei die Konzentration oder Aktivität des malT- und des yaaU-Genprodukts (Proteins) um mindestens 10%, bezogen auf die Aktivität oder Konzentration des Genprodukts in dem Mikroorganismus oder Parentalstamm, der nicht in Bezug auf das malT-Gen und den offenen yaaU-Leserahmen rekombinant ist, durch die Überexpression des malT-Gens und des offenen yaaU-Leserahmens erhöht ist.

13. Mikroorganismen nach Anspruch 1, wobei die Mikroorganismen aus den Gattungen Escherichia, Erwinia, Providencia und Serratia ausgewählt werden.

14. Verfahren zur Herstellung von L-Threonin oder L-Lysin durch Fermentation rekombinanter Mikroorganismen aus der Familie Enterobacteriaceae, wobei
a) die Mikroorganismen, die die gewünschte L-Aminosäure produzieren und in denen das malT-Gen, das für den positiven Transkriptionsaktivator des Maltose-Regulons codiert, und der offene yaaU-Leserahmen von E. coli, der für ein Genprodukt codiert, das als putatives Transportprotein bezeichnet wird, wobei der offene yaaU-Leserahmen das Protein YaaU codiert, das der Zugangs-Nr. AE005181 der Fig. 3 entspricht, oder Nukleotidsequenzen oder Allele, die für die Genprodukte MalT oder YaaU codieren, überexprimiert werden, in einem Medium unter Bedingungen gezüchtet werden, unter denen die gewünschte L-Aminosäure in dem Medium oder in den Zellen konzentriert wird, und
b) die gewünschte L-Aminosäure isoliert wird, wobei Bestandteile der Fermentationsbrühe und/oder der Biomasse in ihrer Gesamtheit oder in Anteilen (≥ 0 bis 100%) davon in dem isolierten Produkt verbleiben oder vollständig entfernt werden.

15. Verfahren nach Anspruch 14, wobei die Mikroorganismen nach den Ansprüchen 1 bis 13 eingesetzt werden.

16. Verfahren nach Anspruch 14 oder 15, wobei zur Herstellung von L-Threonin Mikroorganismen aus der Familie Enterobacteriaceae, in denen zusätzlich zur gleichen Zeit ein oder mehrere der Gene, ausgewählt aus der Gruppe, bestehend aus:
16.1 mindestens einem Gen des thrABC-Operons, das für Aspartatkinase, Homoserin-Dehydrogenase, Homoserinkinase und Threonin-Synthase codiert,
16.2 dem pyc-Gen von Corynebacterium glutamicum, das für Pyruvat-Carboxylase codiert,
16.3 dem pps-Gen, das für Phosphoenolpyruvat-Synthase codiert,
16.4 dem ppc-Gen, das für Phosphoenolpyruvat-Carboxylase codiert,
16.5 den Genen pntA und pntB, die für Untereinheiten der Pyridin-Transhydrogenase codieren,
16.6 dem rhtB-Gen, das für das Protein codiert, das Homoserin-Resistenz verleiht,
16.7 dem rhtC Gen, das für das Protein codiert, das Threonin-Resistenz verleiht,
16.8 dem thrE-Gen von Corynebacterium, das für das Threonin-Exportcarrierprotein codiert,
16.9 dem gdhA-Gen, das für Glutamat-Dehydrogenase codiert,
16.10 dem pgm-Gen, das für Phosphoglucomutase codiert,
16.11 dem fba-Gen, das für Fructosebisphosphat-Aldolase codiert,
16.12 dem ptsH-Gen, das für Phosphohistidinprotein-Hexose-Phosphotransferase codiert,
16.13 dem ptsI-Gen, das für Enzym I des Phosphotransferase-Systems codiert,
16.14 dem crr-Gen,dem für die glucosespezifische IIA-Komponente codiert,
16.15 dem ptsG-Gen, das für die glucosespezifische IIBC-Komponente codiert,
16.16 dem lrp-Gen, das für den Regulator des Leucin-Regulons codiert,
16.17 dem fadR-Gen, das für den Regulator des fad-Regulons codiert,
16.18 dem iclR-Gen, das für den Regulator des zentralen Zwischenproduktstoffwechsels codiert,
16.19 dem ahpC-Gen, das für die kleine Untereinheit der Alkylhydroperoxid-Reduktase codiert,
16.20 dem ahpF-Gen, das für die große Untereinheit der Alkylhydroperoxid-Reduktase codiert,
16.21 dem cysK-Gen, das für Cystein-Synthase A codiert,
16.22 dem cysB-Gen, das für den Regulator des cys-Regulons codiert,
16.23 dem cysJ-Gen, das für das Flavoprotein der NADPH-Sulfit-Reduktase codiert,
16.24 dem cysI-Gen, das für das Hämoprotein der NADPH-Sulfit-Reduktase codiert,
16.25 dem cysH-Gen, das für Adenylylsulfat-Reduktase codiert,
16.26 dem rseA-Gen, das für ein Membranprotein mit Anti-sigmaE-Aktivität codiert,
16.27 dem rseC-Gen, das für einen globalen Regulator des sigmaE-Faktors codiert,
16.28 dem sucA-Gen, das für die Decarboxylase-Untereinheit der 2-Ketoglutarat-Dehydrogenase codiert,
16.29 dem sucB-Gen, das für die Dihydrolipoyltranssuccinase-E2-Untereinheit der 2-Ketoglutarat-Dehydrogenase codiert,
16.30 dem sucC-Gen, das für die β-Untereinheit der Succinyl-CoA-Synthetase codiert,
16.31 dem sucD-Gen, das für die α-Untereinheit der Succinyl-CoA-Synthetase codiert,
16.32 dem aceE-Gen, das für die E1-Komponente des Pyruvat-Dehydrogenase-Komplexes codiert,
16.33 dem aceF-Gen, das für die E2-Komponente des Pyruvat-Dehydrogenase-Komplexes codiert,
16.34 dem rseB-Gen, das für den Regulator der sigmaE-Faktor-Aktivität codiert, und
16.35 dem Genprodukt des offenen Leserahmens (ORF) yodA von Escherichia coli,
überexprimiert wird oder werden, fermentiert werden.

17. Verfahren nach Anspruch 14 oder 15, wobei zur Herstellung von L-Threonin Mikroorganismen aus der Familie Enterobacteriaceae, in denen zusätzlich zur gleichen Zeit ein oder mehrere der Gene, ausgewählt aus der Gruppe, bestehend aus:
17.1 dem tdh-Gen, das für Threonin-Dehydrogenase codiert,
17.2 dem mdh-Gen, das für Malat-Dehydrogenase codiert,
17.3 dem Genprodukt des offenen Leserahmens (ORF) yjfA von Escherichia coli,
17.4 dem Genprodukt des offenen Leserahmens (ORF) ytfP von Escherichia coli,
17.5 dem pckA-Gen, das für Phosphoenolpyruvat-Carboxykinase codiert,
17.6 dem poxB-Gen, das für Pyruvat-Oxidase codiert,
17.7 dem dgsA-Gen, das für den DgsA-Regulator des Phosphotransferase-Systems codiert,
17.8 dem fruR-Gen, das für den Fructose-Repressor codiert,
17.9 dem rpoS-Gen, das für den Sigma³⁸-Faktor codiert, und
17.10 dem aspA-Gen, das für Aspartat-Ammonium-Lyase codiert,
attenuiert oder eliminiert ist oder sind, fermentiert werden.

## Revendications

1. Microorganismes recombinants de la famille des Enterobacteriaceae, qui contiennent un gène malT surexprimé qui code pour l'activateur transcriptionnel positif du régulon maltose, et un cadre ouvert de lecture yaaU surexprimé de E. coli, qui code pour un produit génique annoté comme étant une protéine de transport putative, ledit cadre ouvert de lecture yaaU codant pour la protéine YaaU correspondant au Numéro d'Accession AE005181 de la Figure 3, et qui produisent de la L-thréonine ou de la L-lysine d'une manière améliorée.

2. Microorganismes selon la revendication 1, dans lesquels un polynucléotide qui code pour un polypeptide dont la séquence d'acides aminés est identique à raison d'au moins 90 % avec la séquence d'acides aminés choisie dans le groupe consistant en SEQ ID NO:4 et SEQ ID NO:6, est surexprimé.

3. Microorganismes selon la revendication 2, qui contiennent un polynucléotide surexprimé correspondant au gène malT choisi dans le groupe consistant en :
a) un polynucléotide ayant la séquence nucléotidique SEQ ID NO:3 ou SEQ ID NO:5 ;
b) un polynucléotide ayant une séquence nucléotidique qui correspond à SEQ ID NO:3 ou à SEQ ID NO:5 dans le contexte d'une dégénérescence du code génétique ;
c) une séquence polynucléotidique dont la séquence s'hybride dans des conditions stringentes à la séquence complémentaire de SEQ ID NO:3 ou SEQ ID NO:5 ;
d) un polynucléotide ayant une séquence SEQ ID NO:3 ou SEQ ID NO:5, qui contient des mutants sens à fonction neutre.

4. Microorganismes selon la revendication 2, dans lesquels le polypeptide a une séquence d'acides aminés qui est identique à raison d'au moins 95 % avec l'une des séquences choisies dans le groupe consistant en SEQ ID NO:4 et SEQ ID NO:6.

5. Microorganismes selon la revendication 2, dans lesquels le polypeptide a la séquence d'acides aminés qui a une identité de 100 % avec SEQ ID NO:4 ou SEQ ID NO:6.

6. Microorganismes selon les revendications 1 à 5, qui sont produits par transformation, par transduction ou par conjugaison ou par une combinaison de ces méthodes, avec un vecteur qui contient le gène malT et/ou le cadre ouvert de lecture yaaU, un allèle de ce gène/cadre ouvert de lecture ou des parties de ce dernier, et/ou un promoteur.

7. Microorganismes selon la revendication 6, dans lesquels le nombre de copies du gène malT et/ou du cadre ouvert de lecture yaaU ou des allèles est augmenté d'au moins 1.

8. Microorganismes selon la revendication 7, dans lesquels l'augmentation du nombre de copies du gène malT et/ou du cadre ouvert de lecture yaaU d'au moins 1 est réalisée par intégration du gène/de l'ORF ou des allèles dans le chromosome des microorganismes.

9. Microorganismes selon la revendication 7, dans lesquels l'augmentation du nombre de copies du gène malT et/ou du cadre ouvert de lecture yaaU d'au moins 1 est réalisée par un vecteur à réplication extrachromosomique.

10. Microorganismes selon la revendication 1 ou 2, dans lesquels, pour réaliser la surexpression,
a) la région promotrice et de régulation, ou le site d'attachement du ribosome en amont du gène malT et/ou du cadre ouvert de lecture yaaU, est mutée, ou
b) des cassettes d'expression ou des promoteurs sont incorporés en amont du gène malT et/ou du cadre ouvert de lecture yaaU.

11. Microorganismes selon la revendication 1 ou 2, dans lesquels le gène malT et/ou le cadre ouvert de lecture yaaU est/sont sous le contrôle d'un promoteur qui conduit à une surexpression du gène/de l'ORF.

12. Microorganismes selon les revendications 1 à 11, dans lesquels la concentration ou l'activité du produit génique malT et/ou yaaU (protéine) est augmentée d'au moins 10 % par rapport à l'activité ou à la concentration du produit génique dans le microorganisme ou dans la souche parente qui n'est pas recombinante pour le gène malT et le cadre ouvert de lecture yaaU, par la surexpression du gène malT et du cadre ouvert de lecture yaaU.

13. Microorganismes selon la revendication 1, les microorganismes étant choisis parmi les genres Escherichia, Erwinia, Providencia et Serratia.

14. Procédé de préparation de L-thréonine ou de L-lysine par fermentation de microorganismes recombinants de la famille des Enterobacteriaceae, dans lequel
a) les microorganismes qui produisent l'acide L-aminé souhaité et dans lesquels le gène malT, qui code pour l'activité transcriptionnel positif du régulon maltose, et le cadre ouvert de lecture yaaU de E. coli, qui code pour un produit génique annoté comme étant une protéine de transport putative, ledit cadre ouvert de lecture yaaU codant pour la protéine YaaU correspondant au Numéro d'Accession AE005181 de la Figure 3, ou les séquences nucléotidiques ou les allèles qui codent pour les produits géniques malT ou YaaU, sont surexprimés, sont cultivés dans un milieu dans des conditions dans lesquelles l'acide L-aminé souhaité est concentré dans le milieu ou dans les cellules, et
b) l'acide L-aminé souhaité est isolé, les constituants du bouillon de fermentation et/ou de la biomasse restants, en totalité ou en partie (≥ 0 à 100 %) restant dans le produit qui a été isolé, ou étant entièrement enlevés.

15. Procédé selon la revendication 14, dans lequel on utilise les microorganismes selon les revendications 1 à 13.

16. Procédé selon la revendication 14 ou 15, dans lequel, pour la préparation de L-thréonine, on fermente des microorganismes de la famille des Enterobacteriaceae, dans lesquels, en outre et au même moment, un ou plusieurs des gènes choisis dans le groupe consistant en :
16.1 au moins un gène de l'opéron thrABC, qui code pour l'aspartate kinase, l'homosérine déshydrogénase, l'homosérine kinase et la thréonine synthase,
16.2 le gène pyc de Corynebacterium glutamicum, qui code pour la pyruvate carboxylase,
16.3 le gène pps, qui code pour la phosphoénol pyruvate synthase,
16.4 le gène ppc, qui code pour la phosphoénol pyruvate carboxylase,
16.5 les gènes pntA et pntB, qui codent pour les sous-unités de la pyridine transhydrogénase,
16.6 le gène rhtB, qui code pour la protéine qui confère une résistance à l'homosérine,
16.7 le gène rhtC, qui code pour la protéine qui confère une résistance à la thréonine,
16.8 le gène thrE de Corynebacterium, qui code pour la protéine transporteuse d'exportation de la thréonine,
16.9 le gène gdhA, qui code pour la glutamate déshydrogénase,
16.10 le gène pgm, qui code pour la phosphoglucomutase,
16.11 le gène fba, qui code pour la fructose biphosphate aldolase,
16.12 le gène ptsH, qui code pour la phosphohistidine protéine hexose phosphotransférase,
16.13 le gène ptsI, qui code pour l'enzyme I du système phosphotransférase,
16.14 le gène crr, qui code pour le composant IIA spécifique de glucose,
16.15 le gène ptsG, qui code pour le composant IIBC spécifique de glucose,
16.16 le gène lrp, qui code pour le régulateur du régulon leucine,
16.17 le gène fadR, qui code pour le régulateur du régulon fad,
16.18 le gène iclR, qui code pour le régulateur du métabolisme intermédiaire central,
16.19 le gène ahpC, qui code pour la petite sous-unité de l'alkyl hydroperoxyde réductase,
16.20 le gène ahpF, qui code pour la grande sous-unité de l'alkyl hydroperoxyde réductase,
16.21 le gène cysK, qui code pour la cystéine synthase A,
16.22 le gène cysB, qui code pour le régulateur du régulon cys,
16.23 le gène cysJ, qui code pour la flavoprotéine de la NADPH sulfite réductase,
16.24 le gène cysI, qui code pour l'hémoprotéine de la NADPH sulfite réductase,
16.25 le gène cysH, qui code pour l'adénylyl sulfate réductase,
16.26 le gène rseA, qui code pour une protéine de membrane ayant une activité anti-sigmaE,
16.27 le gène rseC, qui code pour un régulateur global du facteur sigmaE,
16.28 le gène sucA, qui code pour la sous-unité décarboxylase de la 2-cétoglutarate déshydrogénase,
16.29 le gène sucB, qui code pour la sous-unité dihydrolipoyl trans-succinase E2 de la 2-cétoglutarate déshydrogénase,
16.30 le gène sucC, qui code pour la sous-unité β de la succinyl-CoA synthétase,
16.31 le gène sucD, qui code pour la sous-unité α de la succinyl-CoA synthétase,
16.32 le gène aceE, qui code pour le composant E1 du complexe pyruvate déshydrogénase,
16.33 le gène aceF, qui code pour le composant E2 du complexe pyruvate déshydrogénase,
16.34 le gène rseB, qui code pour le régulateur de l'activité du facteur sigmaE, et
16.35 le produit génique du cadre ouvert de lecture (ORF) yodA d'Escherichia coli,
est ou sont surexprimés.

17. Procédé selon la revendication 14 ou 15, dans lequel, pour la préparation de la L-thréonine, on fermente des microorganismes de la famille des Enterobacteriaceae, dans lesquels, en plus, en même temps, un ou plusieurs des gènes choisis dans le groupe consistant en :
17.1 le gène tdh, qui code pour la thréonine déshydrogénase,
17.2 le gène mdh, qui code pour la malate déshydrogénase,
17.3 le produit génique du cadre ouvert de lecture (ORF) yjfA d'Escherichia coli,
17.4 le produit génique du cadre ouvert de lecture (ORF) ytfP d'Escherichia coli,
17.5 le gène pckA, qui code pour la phosphoénol pyruvate carboxykinase,
17.6 le gène poxB, qui code pour la pyruvate oxydase,
17.7 le gène dgsA, qui code pour le régulateur DgsA du système phosphotransférase,
17.8 le gène fruR, qui code pour le répresseur de fructose,
17.9 le gène rpoS, qui code pour le facteur sigma³⁸, et
17.10 le gène aspA, qui code pour l'aspartate ammonium lyase
est ou sont atténués ou éliminés.


### SEQUENCE LISTING

<110> Degussa AG
<120> Process for the preparation of L-amino acids using strains of the Enterobacteriaceae family
<130> 020487 BT
<160> 6
<170> PatentIn version 3.2
<210> 1
   <211> 28
   <212> DNA
   <213> Artificial sequence
<220>
   <221> Primer
   <222> (1)..(28)
   <223> malT1
<400> 1
   cctcattcta gacagtgaag tgattaac 28
<210> 2
   <211> 26
   <212> DNA
   <213> Artificial sequence
<220>
   <221> Primer
   <222> (1).. (26)
   <223> malT2
<400> 2
   ggcgcgttat caagcttaac ttacac 26
<210> 3
   <211> 2755
   <212> DNA
   <213> Echerichia coli
<220>
   <221> PCR product
   <222> (1)..(2755)
<220>
   <221> CDS
   <222> (30)..(2735)
   <223> malT gene
<400> 3
   cctcattcta gacagtgaag tgattaact atg ctg att ccg tca aaa cta agt      53
                                   Met Leu Ile Pro Ser Lys Leu Ser
                                   1               5
   cgt ccg gtt cga ctc gac cat acc gtg gtt cgt gag cgc ctg ctg gct      101
   Arg Pro Val Arg Leu Asp His Thr Val Val Arg Glu Arg Leu Leu Ala
       10                  15                  20
   aaa ctt tcc ggc gcg aac aac ttc cgg ctg gcg ctg atc acg agt cct      149
   Lys Leu Ser Gly Ala Asn Asn Phe Arg Leu Ala Leu Ile Thr Ser Pro
   25                  30                  35                  40
   gcg ggc tac gga aag acc acc ctc att tcc cag tgg gcg gca ggc aaa      197
   Ala Gly Tyr Gly Lys Thr Thr Leu Ile Ser Gln Trp Ala Ala Gly Lys
                   45                  50                  55
   aac gat atc ggc tgg tac tcg ctg gat gaa ggt gat aac cag caa gag      245
   Asn Asp Ile Gly Trp Tyr Ser Leu Asp Glu Gly Asp Asn Gln Gln Glu
               60                  65                  70
   cgt ttc gcc agc tat ctc att gcc gcc gtg cag cag gca acc aac ggt      293
   Arg Phe Ala Ser Tyr Leu Ile Ala Ala Val Gln Gln Ala Thr Asn Gly
           75                  80                  85
   cac tgt gcg ata tgt gag acg atg gcg caa aaa cgg caa tat gcc agc      341
   His Cys Ala Ile Cys Glu Thr Met Ala Gln Lys Arg Gln Tyr Ala Ser
       90                  95                  100
   ctg acg tca ctc ttc gcc cag ctt ttc att gag ctg gcg gaa tgg cat      389
   Leu Thr Ser Leu Phe Ala Gln Leu Phe Ile Glu Leu Ala Glu Trp His
   105                 110                 115                 120
   agc cca ctt tat ctg gtc atc gat gac tat cat ctg atc act aat cca      437
   Ser Pro Leu Tyr Leu Val Ile Asp Asp Tyr His Leu Ile Thr Asn Pro
                   125                 130                 135
   gtg atc cac gag tca atg cgc ttc ttt att cgc cat caa cca gaa aat      485
   Val Ile His Glu Ser Met Arg Phe Phe Ile Arg His Gln Pro Glu Asn
               140                 145                 150
   ctc acc ctg gtg gtg ttg tca cgc aac ctt ccg caa ctg ggc att gcc      533
   Leu Thr Leu Val Val Leu Ser Arg Asn Leu Pro Gln Leu Gly Ile Ala
           155                 160                 165
   aat ctg cgt gtt cgt gat caa ctg ctg gaa att ggc agt cag caa ctg      581
   Asn Leu Arg Val Arg Asp Gln Leu Leu Glu Ile Gly Ser Gln Gln Leu
       170                 175                 180
   gca ttt acc cat cag gaa gcg aag cag ttt ttt gat tgc cgt ctg tca      629
   Ala Phe Thr His Gln Glu Ala Lys Gln Phe Phe Asp Cys Arg Leu Ser
   185                 190                 195                 200
   tcg ccg att gaa gcc gca gaa agc agt cgg att tgc gat gac gtt tcc      677
   Ser Pro Ile Glu Ala Ala Glu Ser Ser Arg Ile Cys Asp Asp Val Ser
                   205                 210                 215
   ggt tgg gcg acg gca cta cag cta atc gcc ctc tcc gcc cgg cag aat      725
   Gly Trp Ala Thr Ala Leu Gln Leu Ile Ala Leu Ser Ala Arg Gln Asn
               220                 225                 230
   acc cac tca gcc cat aag tcg gca cgc cgc ctg gcg gga atc aat gcc      773
   Thr His Ser Ala His Lys Ser Ala Arg Arg Leu Ala Gly Ile Asn Ala
           235                 240                 245
   agc cat ctt tcg gat tat ctg gtc gat gag gtt ttg gat aac gtc gat      821
   Ser His Leu Ser Asp Tyr Leu Val Asp Glu Val Leu Asp Asn Val Asp
       250                 255                 260
   ctc gca acg cgc cat ttt ctg ttg aaa agc gcc att ttg cgc tca atg      869
   Leu Ala Thr Arg His Phe Leu Leu Lys Ser Ala Ile Leu Arg Ser Met
   265                 270                 275                 280
   aac gat gcc ctc atc acc cgt gtg acc ggc gaa gaa aac ggg caa atg      917
   Asn Asp Ala Leu Ile Thr Arg Val Thr Gly Glu Glu Asn Gly Gln Met
                   285                 290                 295
   cgc ctc gaa gag att gag cgt cag ggg ctg ttt tta cag cgg atg gat      965
   Arg Leu Glu Glu Ile Glu Arg Gln Gly Leu Phe Leu Gln Arg Met Asp
               300                 305                 310
   gat acc ggc gag tgg ttc tgc tat cac ccg ctg ttt ggt aac ttc ctg     1013
   Asp Thr Gly Glu Trp Phe Cys Tyr His Pro Leu Phe Gly Asn Phe Leu
           315                 320                 325
   cgc cag cgc tgc cag tgg gaa ctg gcg gcg gag ctg ccg gaa atc cac     1061
   Arg Gln Arg Cys Gln Trp Glu Leu Ala Ala Glu Leu Pro Glu Ile His
       330                 335                 340
   cgt gcc gcc gca gaa agc tgg atg gcc cag gga ttt ccc agc gaa gca     1109
   Arg Ala Ala Ala Glu Ser Trp Met Ala Gln Gly Phe Pro Ser Glu Ala
   345                 350                 355                 360
   att cat cat gcg ctg gcg gca ggc gat gcg ctg atg ctg cgc gat att     1157
   Ile His His Ala Leu Ala Ala Gly Asp Ala Leu Met Leu Arg Asp Ile
                   365                 370                 375
   ctg ctt aat cac gcc tgg agt ctg ttc aac cat agc gaa ctg tcg ctg     1205
   Leu Leu Asn His Ala Trp Ser Leu Phe Asn His Ser Glu Leu Ser Leu
               380                 385                 390
   ctg gaa gag tcg ctt aag gcc ctg ccg tgg gac agc ttg ctg gaa aat     1253
   Leu Glu Glu Ser Leu Lys Ala Leu Pro Trp Asp Ser Leu Leu Glu Asn
           395                 400                 405
   ccg cag ttg gtg tta ttg cag gcg tgg ctg atg caa agc caa cat cgc     1301
   Pro Gln Leu Val Leu Leu Gln Ala Trp Leu Met Gln Ser Gln His Arg
       410                 415                 420
   tac ggc gaa gtt aac acc ctg cta gcc cgt gct gaa cat gaa atc aag     1349
   Tyr Gly Glu Val Asn Thr Leu Leu Ala Arg Ala Glu His Glu Ile Lys
   425                 430                 435                 440
   gac atc aga gaa gac acc atg cac gca gaa ttt aac gct ctg cgc gcc     1397
   Asp Ile Arg Glu Asp Thr Met His Ala Glu Phe Asn Ala Leu Arg Ala
                   445                 450                 455
   cag gtg gcg att aac gat ggt aat ccg gat gaa gcg gaa cgg ctg gca     1445
   Gln Val Ala Ile Asn Asp Gly Asn Pro Asp Glu Ala Glu Arg Leu Ala
               460                 465                 470
   aaa ctg gca ctg gaa gag ctg ccg ccg ggc tgg ttc tat agc cgc att     1493
   Lys Leu Ala Leu Glu Glu Leu Pro Pro Gly Trp Phe Tyr Ser Arg Ile
           475                 480                 485
   gtg gca acc tcg gtg ctg ggt gaa gtg ctg cac tgc aaa ggc gaa ttg     1541
   Val Ala Thr Ser Val Leu Gly Glu Val Leu His Cys Lys Gly Glu Leu
       490                 495                 500
   acc cgc tca ctg gcg cta atg cag caa acc gaa cag atg gca cgc cag     1589
   Thr Arg Ser Leu Ala Leu Met Gln Gln Thr Glu Gln Met Ala Arg Gln
   505                 510                 515                 520
   cac gat gtc tgg cac tac gct ttg tgg agt tta atc cag caa agt gaa     1637
   His Asp Val Trp His Tyr Ala Leu Trp Ser Leu Ile Gln Gln Ser Glu
                   525                     530                     535
   att ctg ttt gcc caa ggg ttc ctg caa acc gcg tgg gaa acg cag gaa     1685
   Ile Leu Phe Ala Gln Gly Phe Leu Gln Thr Ala Trp Glu Thr Gln Glu
               540                     545                     550
   aaa gca ttc cag ctg atc aac gag cag cat ctg gaa cag ctg cca atg     1733
   Lys Ala Phe Gln Leu Ile Asn Glu Gln His Leu Glu Gln Leu Pro Met
           555                     560                     565
   cat gag ttt ctg gtg cgc att cgt gcg cag ctg tta tgg gcc tgg gcg     1781
   His Glu Phe Leu Val Arg Ile Arg Ala Gln Leu Leu Trp Ala Trp Ala
       570                     575                     580
   cgg ctg gat gaa gcc gaa gcg tcg gcg cgt agc ggg att gaa gtc ttg     1829
   Arg Leu Asp Glu Ala Glu Ala Ser Ala Arg Ser Gly Ile Glu Val Leu
   585                     590                     595                     600
   tcg tct tat cag cca cag caa cag ctt cag tgc ctg gca atg ttg att     1877
   Ser Ser Tyr Gln Pro Gln Gln Gln Leu Gln Cys Leu Ala Met Leu Ile
                   605                     610                     615
   caa tgc tcg ctg gcc cgt ggt gat tta gat aac gcc cgt agc cag ctg     1925
   Gln Cys Ser Leu Ala Arg Gly Asp Leu Asp Asn Ala Arg Ser Gln Leu
               620                     625                     630
   aac cgt ctg gaa aac ctg ctg ggg aat ggc aaa tat cac agc gac tgg     1973
   Asn Arg Leu Glu Asn Leu Leu Gly Asn Gly Lys Tyr His Ser Asp Trp
           635                     640                     645
   atc tct aac gcc aac aaa gtc cgg gtg att tac tgg caa atg acc ggc     2021
   Ile Ser Asn Ala Asn Lys Val Arg Val Ile Tyr Trp Gln Met Thr Gly
       650                     655                     660
   gat aaa gcc gcc gct gcc aac tgg ttg cgt cat acg gct aaa cca gag     2069
   Asp Lys Ala Ala Ala Ala Asn Trp Leu Arg His Thr Ala Lys Pro Glu
   665                     670                     675                     680
   ttt gcg aac aac cac ttc ctg caa ggt caa tgg cgc aac att gcc cgt     2117
   Phe Ala Asn Asn His Phe Leu Gln Gly Gln Trp Arg Asn Ile Ala Arg
                   685                     690                     695
   gca caa atc ttg ctg ggc gag ttt gaa ccg gca gaa att gtt ctc gaa     2165
   Ala Gln Ile Leu Leu Gly Glu Phe Glu Pro Ala Glu Ile Val Leu Glu
               700                     705                     710
   gaa ctc aat gaa aat gcc cgg agt ctg cgg ttg atg agc gat ctc aac     2213
   Glu Leu Asn Glu Asn Ala Arg Ser Leu Arg Leu Met Ser Asp Leu Asn
           715                     720                     725
   cgt aac ctg ttg ctg ctt aat caa ctg tac tgg cag gcc gga cgt aaa     2261
   Arg Asn Leu Leu Leu Leu Asn Gln Leu Tyr Trp Gln Ala Gly Arg Lys
       730                     735                     740
   agt gac gcc cag cgc gtg ttg ctg gac gca tta aaa ctg gcg aat cgc     2309
   Ser Asp Ala Gln Arg Val Leu Leu Asp Ala Leu Lys Leu Ala Asn Arg
   745                     750                     755                     760
   acc gga ttt atc agc cat ttt gtc atc gaa ggc gaa gcg atg gcg caa     2357
   Thr Gly Phe Ile Ser His Phe Val Ile Glu Gly Glu Ala Met Ala Gln
                   765                     770                     775
   caa ctg cgt cag ctg att cag ctt aat acg ctg ccg gaa ctg gaa cag      2405
   Gln Leu Arg Gln Leu Ile Gln Leu Asn Thr Leu Pro Glu Leu Glu Gln
               780                 785                 790
   cat cgc gcg cag cgt att ctg cga gaa atc aat caa cat cat cgg cat      2453
   His Arg Ala Gln Arg Ile Leu Arg Glu Ile Asn Gln His His Arg His
           795                 800                 805
   aaa ttc gcc cat ttc gat gag aat ttc gtt gaa cgt ctg cta aat cat      2501
   Lys Phe Ala His Phe Asp Glu Asn Phe Val Glu Arg Leu Leu Asn His
       810                 815                 820
   cct gaa gta cct gaa ctg atc cgc acc agc ccg ctg acg caa cgt gaa      2549
   Pro Glu Val Pro Glu Leu Ile Arg Thr Ser Pro Leu Thr Gln Arg Glu
   825                 830                 835                 840
   tgg cag gta ctg ggg ctg atc tac tct ggt tac agc aat gag caa att      2597
   Trp Gln Val Leu Gly Leu Ile Tyr Ser Gly Tyr Ser Asn Glu Gln Ile
                   845                 850                 855
   gcc gga gaa ctg gaa gtc gcg gca acc acc atc aaa acg cat atc cgc      2645
   Ala Gly Glu Leu Glu Val Ala Ala Thr Thr Ile Lys Thr His Ile Arg
               860                 865                 870
   aat ctg tat cag aaa ctc ggc gtg gcc cat cgc cag gat gcg gta caa      2693
   Asn Leu Tyr Gln Lys Leu Gly Val Ala His Arg Gln Asp Ala Val Gln
           875                 880                 885
   cac gcc cag caa ttg ctg aag atg atg ggg tac ggc gtg taa              2735
   His Ala Gln Gln Leu Leu Lys Met Met Gly Tyr Gly Val
       890                 895                 900
   gttaagcttg ataacgcgcc                                                2755
<210> 4
   <211> 901
   <212> PRT
   <213> Echerichia coli
<400> 4
   Met Leu Ile Pro Ser Lys Leu Ser Arg Pro Val Arg Leu Asp His Thr
   1 5 10 15
   Val Val Arg Glu Arg Leu Leu Ala Lys Leu Ser Gly Ala Asn Asn Phe
   20 25 30
   Arg Leu Ala Leu Ile Thr Ser Pro Ala Gly Tyr Gly Lys Thr Thr Leu
   35 40 45
   Ile Ser Gln Trp Ala Ala Gly Lys Asn Asp Ile Gly Trp Tyr Ser Leu
   50 55 60
   Asp Glu Gly Asp Asn Gln Gln Glu Arg Phe Ala Ser Tyr Leu Ile Ala
   65 70 75 80
   Ala Val Gln Gln Ala Thr Asn Gly His Cys Ala Ile Cys Glu Thr Met
   85 90 95
   Ala Gln Lys Arg Gln Tyr Ala Ser Leu Thr Ser Leu Phe Ala Gln Leu
   100 105 110
   Phe Ile Glu Leu Ala Glu Trp His Ser Pro Leu Tyr Leu Val Ile Asp
   115 120 125
   Asp Tyr His Leu Ile Thr Asn Pro Val Ile His Glu Ser Met Arg Phe
   130 135 140
   Phe Ile Arg His Gln Pro Glu Asn Leu Thr Leu Val Val Leu Ser Arg
   145 150 155 160
   Asn Leu Pro Gln Leu Gly Ile Ala Asn Leu Arg Val Arg Asp Gln Leu
   165 170 175
   Leu Glu Ile Gly Ser Gln Gln Leu Ala Phe Thr His Gln Glu Ala Lys
   180 185 190
   Gln Phe Phe Asp Cys Arg Leu Ser Ser Pro Ile Glu Ala Ala Glu Ser
   195 200 205
   Ser Arg Ile Cys Asp Asp Val Ser Gly Trp Ala Thr Ala Leu Gln Leu
   210 215 220
   Ile Ala Leu Ser Ala Arg Gln Asn Thr His Ser Ala His Lys Ser Ala
   225 230 235 240
   Arg Arg Leu Ala Gly Ile Asn Ala Ser His Leu Ser Asp Tyr Leu Val
   245 250 255
   Asp Glu Val Leu Asp Asn Val Asp Leu Ala Thr Arg His Phe Leu Leu
   260 265 270
   Lys Ser Ala Ile Leu Arg Ser Met Asn Asp Ala Leu Ile Thr Arg Val
   275 280 285
   Thr Gly Glu Glu Asn Gly Gln Met Arg Leu Glu Glu Ile Glu Arg Gln
   290 295 300
   Gly Leu Phe Leu Gln Arg Met Asp Asp Thr Gly Glu Trp Phe Cys Tyr
   305 310 315 320
   His Pro Leu Phe Gly Asn Phe Leu Arg Gln Arg Cys Gln Trp Glu Leu
   325 330 335
   Ala Ala Glu Leu Pro Glu Ile His Arg Ala Ala Ala Glu Ser Trp Met
   340 345 350
   Ala Gln Gly Phe Pro Ser Glu Ala Ile His His Ala Leu Ala Ala Gly
   355 360 365
   Asp Ala Leu Met Leu Arg Asp Ile Leu Leu Asn His Ala Trp Ser Leu
   370 375 380
   Phe Asn His Ser Glu Leu Ser Leu Leu Glu Glu Ser Leu Lys Ala Leu
   385 390 395 400
   Pro Trp Asp Ser Leu Leu Glu Asn Pro Gln Leu Val Leu Leu Gln Ala
   405 410 415
   Trp Leu Met Gln Ser Gln His Arg Tyr Gly Glu Val Asn Thr Leu Leu
   420 425 430
   Ala Arg Ala Glu His Glu Ile Lys Asp Ile Arg Glu Asp Thr Met His
   435 440 445
   Ala Glu Phe Asn Ala Leu Arg Ala Gln Val Ala Ile Asn Asp Gly Asn
   450 455 460
   Pro Asp Glu Ala Glu Arg Leu Ala Lys Leu Ala Leu Glu Glu Leu Pro
   465 470 475 480
   Pro Gly Trp Phe Tyr Ser Arg Ile Val Ala Thr Ser Val Leu Gly Glu
   485 490 495
   Val Leu His Cys Lys Gly Glu Leu Thr Arg Ser Leu Ala Leu Met Gln
   500 505 510
   Gln Thr Glu Gln Met Ala Arg Gln His Asp Val Trp His Tyr Ala Leu
   515 520 525
   Trp Ser Leu Ile Gln Gln Ser Glu Ile Leu Phe Ala Gln Gly Phe Leu
   530 535 540
   Gln Thr Ala Trp Glu Thr Gln Glu Lys Ala Phe Gln Leu Ile Asn Glu
   545 550 555 560
   Gln His Leu Glu Gln Leu Pro Met His Glu Phe Leu Val Arg Ile Arg
   565 570 575
   Ala Gln Leu Leu Trp Ala Trp Ala Arg Leu Asp Glu Ala Glu Ala Ser
   580 585 590
   Ala Arg Ser Gly Ile Glu Val Leu Ser Ser Tyr Gln Pro Gln Gln Gln
   595 600 605
   Leu Gln Cys Leu Ala Met Leu Ile Gln Cys Ser Leu Ala Arg Gly Asp
   610 615 620
   Leu Asp Asn Ala Arg Ser Gln Leu Asn Arg Leu Glu Asn Leu Leu Gly
   625 630 635 640
   Asn Gly Lys Tyr His Ser Asp Trp Ile Ser Asn Ala Asn Lys Val Arg
   645 650 655
   Val Ile Tyr Trp Gln Met Thr Gly Asp Lys Ala Ala Ala Ala Asn Trp
   660 665 670
   Leu Arg His Thr Ala Lys Pro Glu Phe Ala Asn Asn His Phe Leu Gln
   675 680 685
   Gly Gln Trp Arg Asn Ile Ala Arg Ala Gln Ile Leu Leu Gly Glu Phe
   690 695 700
   Glu Pro Ala Glu Ile Val Leu Glu Glu Leu Asn Glu Asn Ala Arg Ser
   705 710 715 720
   Leu Arg Leu Met Ser Asp Leu Asn Arg Asn Leu Leu Leu Leu Asn Gln
   725 730 735
   Leu Tyr Trp Gln Ala Gly Arg Lys Ser Asp Ala Gln Arg Val Leu Leu
   740 745 750
   Asp Ala Leu Lys Leu Ala Asn Arg Thr Gly Phe Ile Ser His Phe Val
   755 760 765
   Ile Glu Gly Glu Ala Met Ala Gln Gln Leu Arg Gln Leu Ile Gln Leu
   770 775 780
   Asn Thr Leu Pro Glu Leu Glu Gln His Arg Ala Gln Arg Ile Leu Arg
   785 790 795 800
   Glu Ile Asn Gln His His Arg His Lys Phe Ala His Phe Asp Glu Asn
   805 810 815
   Phe Val Glu Arg Leu Leu Asn His Pro Glu Val Pro Glu Leu Ile Arg
   820 825 830
   Thr Ser Pro Leu Thr Gln Arg Glu Trp Gln Val Leu Gly Leu Ile Tyr
   835 840 845
   Ser Gly Tyr Ser Asn Glu Gln Ile Ala Gly Glu Leu Glu Val Ala Ala
   850 855 860
   Thr Thr Ile Lys Thr His Ile Arg Asn Leu Tyr Gln Lys Leu Gly Val
   865 870 875 880
   Ala His Arg Gln Asp Ala Val Gln His Ala Gln Gln Leu Leu Lys Met
   885 890 895
   Met Gly Tyr Gly Val
   900
<210> 5
   <211> 2706
   <212> DNA
   <213> Salmonella typhimurium
<220>
   <221> CDS
   <222> (1)..(2706)
   <223> malT coding region
<400> 5
   atg ttg att ccg tct aaa tta agt cgt ccg gtt cgt ctc gac cat acc      48
   Met Leu Ile Pro Ser Lys Leu Ser Arg Pro Val Arg Leu Asp His Thr
   1               5                   10                  15
   gtg gtt cgt gag cgg ctg ttg gct aaa ctt tcc ggc gcg aac aat ttc      96
   Val Val Arg Glu Arg Leu Leu Ala Lys Leu Ser Gly Ala Asn Asn Phe
               20                  25                  30
   agg ctc gcc ctg gta acc agt cct gct ggc tat ggg aaa acc acg ctt     144
   Arg Leu Ala Leu Val Thr Ser Pro Ala Gly Tyr Gly Lys Thr Thr Leu
           35                  40                  45
   gtc tca cag tgg gcg gcg ggc aaa aat gaa tta ggc tgg tac tct ctc     192
   Val Ser Gln Trp Ala Ala Gly Lys Asn Glu Leu Gly Trp Tyr Ser Leu
       50                  55                  60
   gac gaa ggc gat aac cag caa gag cga ttc gcc agt tac tta att gcc     240
   Asp Glu Gly Asp Asn Gln Gln Glu Arg Phe Ala Ser Tyr Leu Ile Ala
   65                  70                  75                  80
   gct atc caa cag gcc act ggc gga cac tgc tcg acc agc gaa gcg atg     288
   Ala Ile Gln Gln Ala Thr Gly Gly His Cys Ser Thr Ser Glu Ala Met
                   85                  90                  95
   gcg caa aaa cgt cag tac gcc agc ctg acg tct ctc ttc gca cag ctc     336
   Ala Gln Lys Arg Gln Tyr Ala Ser Leu Thr Ser Leu Phe Ala Gln Leu
               100                 105                 110
   ttt att gaa ctg gcg cag tgg cat cgc ccg ctt tat ctg gtg att gac     384
   Phe Ile Glu Leu Ala Gln Trp His Arg Pro Leu Tyr Leu Val Ile Asp
           115                 120                 125
   gac tac cat ttg att acc aat ccg gtg att cac gac gcg atg cgc ttt     432
   Asp Tyr His Leu Ile Thr Asn Pro Val Ile His Asp Ala Met Arg Phe
       130                 135                 140
   ttc tta cgg cat cag ccg gaa aat ttc acc ctg gtg gta tta tcg cgt     480
   Phe Leu Arg His Gln Pro Glu Asn Phe Thr Leu Val Val Leu Ser Arg
   145                 150                 155                 160
   aat ttg ccg caa ctg ggc atc gct aac ctg cgc gtg cgc gat cag ctg      528
   Asn Leu Pro Gln Leu Gly Ile Ala Asn Leu Arg Val Arg Asp Gln Leu
               165                 170                 175
   ttg gaa atc ggc agc cag cag ttg gct ttt aac cat cag gaa gca aag      576
   Leu Glu Ile Gly Ser Gln Gln Leu Ala Phe Asn His Gln Glu Ala Lys
           180                 185                 190
   caa ttt ttt gac cgt cgt ctt tcg tcg ccc att gaa gcc gct gag agc      624
   Gln Phe Phe Asp Arg Arg Leu Ser Ser Pro Ile Glu Ala Ala Glu Ser
       195                 200                 205
   agc cgc atg tgc gac gat gtg gca ggt tgg gcc act gcg cta cag ttg      672
   Ser Arg Met Cys Asp Asp Val Ala Gly Trp Ala Thr Ala Leu Gln Leu
       210                 215                 220
   atc gcc ctc tcc gcg cgc cag aac cat acc tct gcg cat cac tcc gcc      720
   Ile Ala Leu Ser Ala Arg Gln Asn His Thr Ser Ala His His Ser Ala
   225                 230                 235                 240
   cgc cgt ctt gcc ggg att aat gcc agc cac ctt tct gat tat ctg gtg      768
   Arg Arg Leu Ala Gly Ile Asn Ala Ser His Leu Ser Asp Tyr Leu Val
                   245                 250                 255
   gac gaa gta ctg gat aac gtg gat gtc agc acc cga cat ttt tta ctt      816
   Asp Glu Val Leu Asp Asn Val Asp Val Ser Thr Arg His Phe Leu Leu
               260                 265                 270
   aaa agc gcg atc tta cgc tca atg aat gac gcg ttg att gtg cgc gtg      864
   Lys Ser Ala Ile Leu Arg Ser Met Asn Asp Ala Leu Ile Val Arg Val
           275                 280                 285
   acc ggc gaa gaa aac ggc cag atg cgg ctg gag gag att gag cgc cag      912
   Thr Gly Glu Glu Asn Gly Gln Met Arg Leu Glu Glu Ile Glu Arg Gln
       290                 295                 300
   ggg tta ttc ctg caa cgc atg gac gac acg ggc gaa tgg ttc agc tat      960
   Gly Leu Phe Leu Gln Arg Met Asp Asp Thr Gly Glu Trp Phe Ser Tyr
   305                 310                 315                 320
   cac ccg ttg ttt ggt agc ttt ctg cgc cag cgt tgc cag tgg gaa ctg     1008
   His Pro Leu Phe Gly Ser Phe Leu Arg Gln Arg Cys Gln Trp Glu Leu
                   325                 330                 335
   gcg gcg gaa ctg ccg gag atc cac cgt gcg gcc gcg gaa agc tgg atg     1056
   Ala Ala Glu Leu Pro Glu Ile His Arg Ala Ala Ala Glu Ser Trp Met
               340                 345                 350
   gaa caa ggt ttt ccc agc gaa gct ata cat cat gcg ctg gcg gcg ggt     1104
   Glu Gln Gly Phe Pro Ser Glu Ala Ile His His Ala Leu Ala Ala Gly
           355                 360                 365
   gac gcg caa atg ctg cgc gat atc ctg ctt aac cat gcg tgg ggg ctg     1152
   Asp Ala Gln Met Leu Arg Asp Ile Leu Leu Asn His Ala Trp Gly Leu
       370                 375                 380
   ttt aat cac agt gaa ctg gcg ctg ctg gaa gag tca tta aaa gcg ctg     1200
   Phe Asn His Ser Glu Leu Ala Leu Leu Glu Glu Ser Leu Lys Ala Leu
   385                 390                 395                 400
   ccg tgg gaa agc ctg ctg gaa aat ccg cgg ctg gtg ttg tta cag gcc     1248
   Pro Trp Glu Ser Leu Leu Glu Asn Pro Arg Leu Val Leu Leu Gln Ala
                   405                 410                 415
   tgg ctg atg caa agc caa cac cgc tac agc gaa gtg aat acg ctg ctg      1296
   Trp Leu Met Gln Ser Gln His Arg Tyr Ser Glu Val Asn Thr Leu Leu
               420                 425                 430
   gcg cgc gcc gag caa gag atc aaa ggg gtc atg gac ggc aca ctg cat      1344
   Ala Arg Ala Glu Gln Glu Ile Lys Gly Val Met Asp Gly Thr Leu His
           435                 440                 445
   gct gaa ttt aac gcg ctg cgc gcc cag gtc gcc atc aac gac ggc aat      1392
   Ala Glu Phe Asn Ala Leu Arg Ala Gln Val Ala Ile Asn Asp Gly Asn
       450                 455                 460
   ccg gaa gag gct gag cgg ctg gcg aaa ctg gcg ctg gat gag ctt cct      1440
   Pro Glu Glu Ala Glu Arg Leu Ala Lys Leu Ala Leu Asp Glu Leu Pro
   465                 470                 475                 480
   ctc gcc tgg ttt tat agc cgt att gtc gcg acc tcc gtt cac ggt gaa      1488
   Leu Ala Trp Phe Tyr Ser Arg Ile Val Ala Thr Ser Val His Gly Glu
                   485                 490                 495
   gtg ctg cac tgt aaa ggc gat ctc agc cag tct tta tcg ctg atg cag      1536
   Val Leu His Cys Lys Gly Asp Leu Ser Gln Ser Leu Ser Leu Met Gln
               500                 505                 510
   caa acc gaa caa atg gcg cgt cat cat gac gtc tgg cat tac gcg ctg      1584
   Gln Thr Glu Gln Met Ala Arg His His Asp Val Trp His Tyr Ala Leu
           515                 520                 525
   tgg agc tta att caa caa agc gaa att cag ttc gcc cag gga ttc ctg      1632
   Trp Ser Leu Ile Gln Gln Ser Glu Ile Gln Phe Ala Gln Gly Phe Leu
       530                 535                 540
   cag gcc gca tgg gaa acg cag gag aga gcc ttc cag ttg atc aaa gag      1680
   Gln Ala Ala Trp Glu Thr Gln Glu Arg Ala Phe Gln Leu Ile Lys Glu
   545                 550                 555                 560
   caa cat ctg gaa caa ctg ccg atg cat gag ttt ctg gta cgt atc cgc      1728
   Gln His Leu Glu Gln Leu Pro Met His Glu Phe Leu Val Arg Ile Arg
                   565                 570                 575
   gcg caa ttg ctg tgg gcg tgg gcg cgc ctt gac gaa gcg gag gcc tcg      1776
   Ala Gln Leu Leu Trp Ala Trp Ala Arg Leu Asp Glu Ala Glu Ala Ser
               580                 585                 590
   gcg cgc agc ggt atc gct gtg ctg tcc acc ttc cag ccg caa caa cag      1824
   Ala Arg Ser Gly Ile Ala Val Leu Ser Thr Phe Gln Pro Gln Gln Gln
           595                 600                 605
   ttg cag tgc ctg acg tta ctg gtg cag tgc tcg ctg gcg cgc ggc gat      1872
   Leu Gln Cys Leu Thr Leu Leu Val Gln Cys Ser Leu Ala Arg Gly Asp
       610                 615                 620
   ctg gat aat gcc cgt agt cag ctt aac cga ctg gaa aat tta tta ggc      1920
   Leu Asp Asn Ala Arg Ser Gln Leu Asn Arg Leu Glu Asn Leu Leu Gly
   625                 630                 635                 640
   aat ggc cgt tat cac tgc gac tgg atc tct aac gcc gat aag gtg cgt      1968
   Asn Gly Arg Tyr His Cys Asp Trp Ile Ser Asn Ala Asp Lys Val Arg
                   645                 650                 655
   gtc att tac tgg cag tta acc ggc gac aaa aaa tcc gcc gcc aac tgg      2016
   Val Ile Tyr Trp Gln Leu Thr Gly Asp Lys Lys Ser Ala Ala Asn Trp
               660                 665                 670
   tta cgt cat acg ccc aaa ccg gcg ttt gcc aat aac cat ttt ctg caa      2064
   Leu Arg His Thr Pro Lys Pro Ala Phe Ala Asn Asn His Phe Leu Gln
           675                 680                 685
   ggg caa tgg cgg aat atc gcc cgc gcg cag atc ctg ctg ggc gaa ttc      2112
   Gly Gln Trp Arg Asn Ile Ala Arg Ala Gln Ile Leu Leu Gly Glu Phe
       690                 695                 700
   gag cct gcg gaa atc gtg ctg gaa gag tta aat gag aat gcc cgt agc      2160
   Glu Pro Ala Glu Ile Val Leu Glu Glu Leu Asn Glu Asn Ala Arg Ser
   705                 710                 715                 720
   ctg cgc tta atg agc gat tta aac tgc aat ctg ctg tta ctc aat cag      2208
   Leu Arg Leu Met Ser Asp Leu Asn Cys Asn Leu Leu Leu Leu Asn Gln
                   725                 730                 735
   ctt tac tgg caa tcc ggg cgg aaa aac gac gcc cag cgc gta ttg ctt      2256
   Leu Tyr Trp Gln Ser Gly Arg Lys Asn Asp Ala Gln Arg Val Leu Leu
               740                 745                 750
   gat gcc tta cag ctc gcc aac cgc acc gga ttt atc agc cat ttt gtg      2304
   Asp Ala Leu Gln Leu Ala Asn Arg Thr Gly Phe Ile Ser His Phe Val
           755                 760                 765
   att gaa ggc gaa gcc atg gcg caa caa ttg cgt cag ctt atc cag ctc      2352
   Ile Glu Gly Glu Ala Met Ala Gln Gln Leu Arg Gln Leu Ile Gln Leu
       770                 775                 780
   aat acg ctg ccg gag atg gag cag cat cgc gcg cag cgc atc ctg cga      2400
   Asn Thr Leu Pro Glu Met Glu Gln His Arg Ala Gln Arg Ile Leu Arg
   785                 790                 795                 800
   gag att aac caa cat cac cga cat aaa ttc gcc cat ttt gac gaa ggc      2448
   Glu Ile Asn Gln His His Arg His Lys Phe Ala His Phe Asp Glu Gly
                   805                 810                 815
   ttc gtc gag cgc ctg ctt aac cat ccg gat gtc ccg gag ctt att cgc      2496
   Phe Val Glu Arg Leu Leu Asn His Pro Asp Val Pro Glu Leu Ile Arg
               820                 825                 830
   acc agc ccg ctc acc cag cgt gaa tgg cag gtg tta ggg ctt atc tat      2544
   Thr Ser Pro Leu Thr Gln Arg Glu Trp Gln Val Leu Gly Leu Ile Tyr
           835                 840                 845
   tcc ggt tac agc aac gaa cag att gcc ggc gaa ctg gcg gtg gcc gcg      2592
   Ser Gly Tyr Ser Asn Glu Gln Ile Ala Gly Glu Leu Ala Val Ala Ala
       850                 855                 860
   acc acc att aag acg cat atc cgc aac ctc tac cag aaa ctc ggc gtc      2640
   Thr Thr Ile Lys Thr His Ile Arg Asn Leu Tyr Gln Lys Leu Gly Val
   865                 870                 875                 880
   gcg cac cgt cag gac gcg gta cag cac gcg cag cag ttg ctg aag atg      2688
   Ala His Arg Gln Asp Ala Val Gln His Ala Gln Gln Leu Leu Lys Met
                   885                 890                 895
   atg ggg tac ggg gtt tga                                              2706
   Met Gly Tyr Gly Val
               900
<210> 6
   <211> 901
   <212> PRT
   <213> Salmonella typhimurium
<400> 6
   Met Leu Ile Pro Ser Lys Leu Ser Arg Pro Val Arg Leu Asp His Thr
   1               5                   10                  15
   Val Val Arg Glu Arg Leu Leu Ala Lys Leu Ser Gly Ala Asn Asn Phe
               20                  25                  30
   Arg Leu Ala Leu Val Thr Ser Pro Ala Gly Tyr Gly Lys Thr Thr Leu
           35                  40                  45
   Val Ser Gln Trp Ala Ala Gly Lys Asn Glu Leu Gly Trp Tyr Ser Leu
       50                  55                  60
   Asp Glu Gly Asp Asn Gln Gln Glu Arg Phe Ala Ser Tyr Leu Ile Ala
   65                  70                  75                  80
   Ala Ile Gln Gln Ala Thr Gly Gly His Cys Ser Thr Ser Glu Ala Met
                   85                  90                  95
   Ala Gln Lys Arg Gln Tyr Ala Ser Leu Thr Ser Leu Phe Ala Gln Leu
               100                 105                 110
   Phe Ile Glu Leu Ala Gln Trp His Arg Pro Leu Tyr Leu Val Ile Asp
           115                 120                 125
   Asp Tyr His Leu Ile Thr Asn Pro Val Ile His Asp Ala Met Arg Phe
       130                 135                 140
   Phe Leu Arg His Gln Pro Glu Asn Phe Thr Leu Val Val Leu Ser Arg
   145                 150                 155                 160
   Asn Leu Pro Gln Leu Gly Ile Ala Asn Leu Arg Val Arg Asp Gln Leu
                   165                 170                 175
   Leu Glu Ile Gly Ser Gln Gln Leu Ala Phe Asn His Gln Glu Ala Lys
               180                 185                 190
   Gln Phe Phe Asp Arg Arg Leu Ser Ser Pro Ile Glu Ala Ala Glu Ser
           195                 200                 205
   Ser Arg Met Cys Asp Asp Val Ala Gly Trp Ala Thr Ala Leu Gln Leu
       210                 215                 220
   Ile Ala Leu Ser Ala Arg Gln Asn His Thr Ser Ala His His Ser Ala
   225                 230                 235                 240
   Arg Arg Leu Ala Gly Ile Asn Ala Ser His Leu Ser Asp Tyr Leu Val
                   245                 250                 255
   Asp Glu Val Leu Asp Asn Val Asp Val Ser Thr Arg His Phe Leu Leu
               260                 265                 270
   Lys Ser Ala Ile Leu Arg Ser Met Asn Asp Ala Leu Ile Val Arg Val
           275                 280                 285
   Thr Gly Glu Glu Asn Gly Gln Met Arg Leu Glu Glu Ile Glu Arg Gln
       290                 295                 300
   Gly Leu Phe Leu Gln Arg Met Asp Asp Thr Gly Glu Trp Phe Ser Tyr
   305                 310                 315                 320
   His Pro Leu Phe Gly Ser Phe Leu Arg Gln Arg Cys Gln Trp Glu Leu
                   325                 330                 335
   Ala Ala Glu Leu Pro Glu Ile His Arg Ala Ala Ala Glu Ser Trp Met
               340                 345                 350
   Glu Gln Gly Phe Pro Ser Glu Ala Ile His His Ala Leu Ala Ala Gly
           355                 360                 365
   Asp Ala Gln Met Leu Arg Asp Ile Leu Leu Asn His Ala Trp Gly Leu
       370                 375                 380
   Phe Asn His Ser Glu Leu Ala Leu Leu Glu Glu Ser Leu Lys Ala Leu
   385                 390                 395                 400
   Pro Trp Glu Ser Leu Leu Glu Asn Pro Arg Leu Val Leu Leu Gln Ala
                   405                 410                 415
   Trp Leu Met Gln Ser Gln His Arg Tyr Ser Glu Val Asn Thr Leu Leu
               420                 425                 430
   Ala Arg Ala Glu Gln Glu Ile Lys Gly Val Met Asp Gly Thr Leu His
           435                 440                 445
   Ala Glu Phe Asn Ala Leu Arg Ala Gln Val Ala Ile Asn Asp Gly Asn
       450                 455                 460
   Pro Glu Glu Ala Glu Arg Leu Ala Lys Leu Ala Leu Asp Glu Leu Pro
   465                 470                 475                 480
   Leu Ala Trp Phe Tyr Ser Arg Ile Val Ala Thr Ser Val His Gly Glu
                   485                 490                 495
   Val Leu His Cys Lys Gly Asp Leu Ser Gln Ser Leu Ser Leu Met Gln
               500                 505                 510
   Gln Thr Glu Gln Met Ala Arg His His Asp Val Trp His Tyr Ala Leu
           515                 520                 525
   Trp Ser Leu Ile Gln Gln Ser Glu Ile Gln Phe Ala Gln Gly Phe Leu
       530                 535                 540
   Gln Ala Ala Trp Glu Thr Gln Glu Arg Ala Phe Gln Leu Ile Lys Glu
   545                 550                 555                 560
   Gln His Leu Glu Gln Leu Pro Met His Glu Phe Leu Val Arg Ile Arg
                   565                 570                 575
   Ala Gln Leu Leu Trp Ala Trp Ala Arg Leu Asp Glu Ala Glu Ala Ser
               580                 585                 590
   Ala Arg Ser Gly Ile Ala Val Leu Ser Thr Phe Gln Pro Gln Gln Gln
           595                 600                 605
   Leu Gln Cys Leu Thr Leu Leu Val Gln Cys Ser Leu Ala Arg Gly Asp
       610                 615                 620
   Leu Asp Asn Ala Arg Ser Gln Leu Asn Arg Leu Glu Asn Leu Leu Gly
   625                 630                 635                 640
   Asn Gly Arg Tyr His Cys Asp Trp Ile Ser Asn Ala Asp Lys Val Arg
                   645                 650                 655
   Val Ile Tyr Trp Gln Leu Thr Gly Asp Lys Lys Ser Ala Ala Asn Trp
               660                 665                 670
   Leu Arg His Thr Pro Lys Pro Ala Phe Ala Asn Asn His Phe Leu Gln
           675                 680                 685
   Gly Gln Trp Arg Asn Ile Ala Arg Ala Gln Ile Leu Leu Gly Glu Phe
       690                 695                 700
   Glu Pro Ala Glu Ile Val Leu Glu Glu Leu Asn Glu Asn Ala Arg Ser
   705                 710                 715                 720
   Leu Arg Leu Met Ser Asp Leu Asn Cys Asn Leu Leu Leu Leu Asn Gln
                   725                 730                 735
   Leu Tyr Trp Gln Ser Gly Arg Lys Asn Asp Ala Gln Arg Val Leu Leu
               740                 745                 750
   Asp Ala Leu Gln Leu Ala Asn Arg Thr Gly Phe Ile Ser His Phe Val
           755                 760                 765
   Ile Glu Gly Glu Ala Met Ala Gln Gln Leu Arg Gln Leu Ile Gln Leu
       770                 775                 780
   Asn Thr Leu Pro Glu Met Glu Gln His Arg Ala Gln Arg Ile Leu Arg
   785                 790                 795                 800
   Glu Ile Asn Gln His His Arg His Lys Phe Ala His Phe Asp Glu Gly
                   805                 810                 815
   Phe Val Glu Arg Leu Leu Asn His Pro Asp Val Pro Glu Leu Ile Arg
               820                 825                 830
   Thr Ser Pro Leu Thr Gln Arg Glu Trp Gln Val Leu Gly Leu Ile Tyr
           835                 840                 845
   Ser Gly Tyr Ser Asn Glu Gln Ile Ala Gly Glu Leu Ala Val Ala Ala
       850                 855                 860
   Thr Thr Ile Lys Thr His Ile Arg Asn Leu Tyr Gln Lys Leu Gly Val
   865                 870                 875                 880
   Ala His Arg Gln Asp Ala Val Gln His Ala Gln Gln Leu Leu Lys Met
                   885                 890                 895
   Met Gly Tyr Gly Val
               900